# EUROPEAN PATENT SPECIFICATION

(11) **EP 1 701 730 B1**
(45) Date of publication and mention of the grant of the patent: **21.08.2013**
(21) Application number: 04801601.8
(22) Date of filing: 09.12.2004
(51) Int. Cl.: A61K 38/00, A61K 35/12, A61K 39/00, C12N 5/02, A61P 25/18, A61P 25/24

(54) **METHOD AND VACCINE COMPRISING COPOLYMER 1 FOR TREATMENT OF PSYCHIATRIC DISORDERS**
VERFAHREN UND VAKZINE MIT COPOLYMER-1 FÜR DIE BEHANDLUNG VON PSYCHIATRISCHEN ERKRANKUNGEN
METHODE ET VACCIN COMPRENANT LE COPOLYMERE 1 POUR LE TRAITEMENT DE TROUBLES PSYCHIATRIQUES

(30) Priority: 09.12.2003 US 527763 P
(43) Date of publication of application: 20.09.2006
(73) Proprietor: YEDA RESEARCH AND DEVELOPMENT CO., LTD., 76100 Rehovot (IL)
(72) Inventor: EISENBACH-SCHWARTZ, Michal, 76353 Rehovot (IL); KIPNIS, Jonathan, 71700 Modiin (IL)
(74) Representative: Vossius & Partner
(86) International application number: PCT/IL2004/001115
(87) International publication number: WO 2005/056574

(56) References cited:
- WO-A1-2005/046719
- WO-A2-02/059143
- US-A1- 2002 037 848
- US-A1- 2003 064 915
- KIPNIS J ET AL: "DUAL ACTION OF GLATIRAMER ACETATE (COP-1) IN THE TREATMENT OF CNS AUTOIMMUNE AND NEURODEGENERATIVE DISORDERS" 1 July 2002 (2002-07-01), TRENDS IN MOLECULAR MEDICINE, XX, XX, PAGE(S) 319 - 323 , XP001205939 * the whole document *
- SCHWARTZ M.: 'Harnessing the Immune System for Neuroprotection: Therapeutic Vaccines for Acute and Chronic Neurodegenerative Disorders' CELL. MOL. NEUROBIOL. vol. 21, no. 6, December 2001, pages 617 - 627, XP001205938
- KIPNIS J. ET AL: 'T Cell Immunity to Copolymer 1 Confers Neuroprotection on the Damaged Optic Nerve: Possible Therapy for Optic Neuropathies' PROC. NATL. ACAD. SCI. vol. 97, no. 13, June 2000, pages 7446 - 7451, XP001019237
- KIPNIS J. AND SCHWARTZ M.: 'Dual Action of Glatiramer Acetate (Cop-1) in the Treatment of CNS Autoimmune andNeurodegenerative Disorders' TRENDS MOL. MED. vol. 8, no. 7, July 2002, pages 319 - 323, XP001205939
- FEINSTEIN A.: 'Multiple Sclerosis, Disease Modifying Treatments and Depression: A Critical Methodological Review' MULT. SCLER. vol. 6, no. 5, October 2000, pages 343 - 348, XP008059830
- ROTHERMUNDT M. ET AL: 'Review of Immunological and Immunopathological Findings in Schizophrenia' vol. 15, no. 4, December 2001, pages 319 - 339, XP002994753
- TEITELBAUM D. ET AL: 'Copolymer 1: From Basic Research to Clinical Application' CELL. MOL. LIFE SCI. vol. 53, no. 1, January 1997, pages 24 - 28, XP002994754
- ULMER J.B. ET AL: 'Enhancement of DNA Vaccine Potency Using Conventional Aluminum Adjuvants' VACCINE vol. 18, no. 1-2, 20 August 1999, pages 18 - 28, XP004178811
- SELA M.: 'Structural Components Responsible for Peptide Antigenicity' APPL. BIOCHEM. BIOTECHNOL. vol. 83, no. 1-3, January 2000 - March 2000, pages 63 - 70, XP008059831

### SEQUENCE LISTING

<110> YEDA RESEARCH AND DEVELOPMENT C0. LTD. EISENBACH-SHWARTZ, Michal KIPNIS, Jonathan
<120> METHOD AND VACCINE COMPRISING COPOLYMER 1 FOR TREATMENT OF PSYCHIATRIC DISORDERS
<130> YEDA-053 PCT
<150> US 60/527,763
   <151> 2003-12-09
<160> 32
<170> PatentIn version 3.2
<210> 1
   <211> 15
   <212> PRT
   <213> Artificial Sequence
<220>
   <223> Synthetic peptide
<400> 1
   Ala Ala Ala Tyr Ala Ala Ala Ala Ala Ala Lys Ala Ala Ala Ala
   1               5                   10                  15
<210> 2
   <211> 15
   <212> PRT
   <213> Artificial Sequence
<220>
   <223> Synthetic peptide
<400> 2
   Ala Glu Lys Tyr Ala Ala Ala Ala Ala Ala Lys Ala Ala Ala Ala
   1               5                   10                  15
<210> 3
   <211> 15
   <212> PRT
   <213> Artificial sequence
<220>
   <223> Synthetic peptide
<400> 3
   Ala Lys Glu Tyr Ala Ala Ala Ala Ala Ala Lys Ala Ala Ala Ala
   1               5                   10                  15
<210> 4
   <211> 15
   <212> PRT
   <213> Artificial Sequence
<220>
   <223> Synthetic peptide
<400> 4
   Ala Lys Lys Tyr Ala Ala Ala Ala Ala Ala Lys Ala Ala Ala Ala
   1 5 10 15
<210> 5
   <211> 15
   <212> PRT
   <213> Artificial sequence
<220>
   <223> synthetic peptide
<400> 5
   Ala Glu Ala Tyr Ala Ala Ala Ala Ala Ala Lys Ala Ala Ala Ala
   1 5 10 15
<210> 6
   <211> 15
   <212> PRT
   <213> Artificial sequence
<220>
   <223> Synthetic peptide
<400> 6
   Lys Glu Ala Tyr Ala Ala Ala Ala Ala Ala Lys Ala Ala Ala Ala
   1 5 10 15
<210> 7
   <211> 15
   <212> PRT
   <213> Artificial Sequence
<220>
   <223> Synthetic peptide
<400> 7
   Ala Glu Glu Tyr Ala Ala Ala Ala Ala Ala Lys Ala Ala Ala Ala
   1 5 10 15
<210> 8
   <211> 15
   <212> PRT
   <213> Artificial sequence
<220>
   <223> Synthetic peptide
<400> 8
   Ala Ala Glu Tyr Ala Ala Ala Ala Ala Ala Lys Ala Ala Ala Ala
   1 5 10 15
<210> 9
   <211> 15
   <212> PRT
   <213> Artificial Sequence
<220>
   <223> Synthetic peptide
<400> 9
   Glu Lys Ala Tyr Ala Ala Ala Ala Ala Ala Lys Ala Ala Ala Ala
   1 5 10 15
<210> 10
   <211> 15
   <212> PRT
   <213> Artificial sequence
<220>
   <223> Synthetic peptide
<400> 10
   Ala Ala Lys Tyr Glu Ala Ala Ala Ala Ala Lys Ala Ala Ala Ala
   1 5 10 15
<210> 11
   <211> 15
   <212> PRT
   <213> Artificial Sequence
<220>
   <223> Synthetic peptide
<400> 11
   Ala Ala Lys Tyr Ala Glu Ala Ala Ala Ala Lys Ala Ala Ala Ala
   1 5 10 15
<210> 12
   <211> 15
   <212> PRT
   <213> Artificial sequence
<220>
   <223> Synthetic peptide
<400> 12
   Glu Ala Ala Tyr Ala Ala Ala Ala Ala Ala Lys Ala Ala Ala Ala
   1 5 10 15
<210> 13
   <211> 15
   <212> PRT
   <213> Artificial Sequence
<220>
   <223> Synthetic peptide
<400> 13
   Glu Lys Lys Tyr Ala Ala Ala Ala Ala Ala Lys Ala Ala Ala Ala
   1 5 10 15
<210> 14
   <211> 15
   <212> PRT
   <213> Artificial sequence
<220>
   <223> Synthetic peptide
<400> 14
   Glu Ala Lys Tyr Ala Ala Ala Ala Ala Ala Lys Ala Ala Ala Ala
   1 5 10 15
<210> 15
   <211> 15
   <212> PRT
   <213> Artificial Sequence
<220>
   <223> Synthetic peptide
<400> 15
   Ala Glu Lys Tyr Ala Ala Ala Ala Ala Ala Ala Ala Ala Ala Ala
   1 5 10 15
<210> 16
   <211> 15
   <212> PRT
   <213> Artificial Sequence
<220>
   <223> synthetic peptide
<400> 16
   Ala Lys Glu Tyr Ala Ala Ala Ala Ala Ala Ala Ala Ala Ala Ala
   1 5 10 15
<210> 17
   <211> 15
   <212> PRT
   <213> Artificial Sequence
<220>
   <223> Synthetic peptide
<400> 17
   Ala Lys Lys Tyr Glu Ala Ala Ala Ala Ala Ala Ala Ala Ala Ala
   1 5 10 15
<210> 18
   <211> 15
   <212> PRT
   <213> Artificial Sequenece
<220>
   <223> Synthetic peptide
<400> 18
   Ala Lys Lys Tyr Ala Glu Ala Ala Ala Ala Ala Ala Ala Ala Ala
   1 5 10 15
<210> 19
   <211> 15
   <212> PRT
   <213> Artificial sequence
<220>
   <223> Synthetic peptide
<400> 19
   Ala Glu Ala Tyr Lys Ala Ala Ala Ala Ala Ala Ala Ala Ala Ala
   1 5 10 15
<210> 20
   <211> 15
   <212> PRT
   <213> Artificial sequence
<220>
   <223> Synthetic peptide
<400> 20
   Lys Glu Ala Tyr Ala Ala Ala Ala Ala Ala Ala Ala Ala Ala Ala
   1 5 10 15
<210> 21
   <211> 15
   <212> PRT
   <213> Artificial Sequence
<220>
   <223> Synthetic peptide
<400> 21
   Ala Glu Glu Tyr Lys Ala Ala Ala Ala Ala Ala Ala Ala Ala Ala
   1 5 10 15
<210> 22
   <211> 15
   <212> PRT
   <213> Artificial sequence
<220>
   <223> Synthetic peptide
<400> 22
   Ala Ala Glu Tyr Lys Ala Ala Ala Ala Ala Ala Ala Ala Ala Ala
   1 5 10 15
<210> 23
   <211> 15
   <212> PRT
   <213> Artificial sequence
<220>
   <223> Synthetic peptide
<400> 23
   Glu Lys Ala Tyr Ala Ala Ala Ala Ala Ala Ala Ala Ala Ala Ala
   1 5 10 15
<210> 24
   <211> 15
   <212> PRT
   <213> Artificial Sequence
<220>
   <223> Synthetic peptide
<400> 24
   Ala Ala Lys Tyr Glu Ala Ala Ala Ala Ala Ala Ala Ala Ala Ala
   1 5 10 15
<210> 25
   <211> 15
   <212> PRT
   <213> Artificial Sequence
<220>
   <223> Synthetic peptide
<400> 25
   Ala Ala Lys Tyr Ala Glu Ala Ala Ala Ala Ala Ala Ala Ala Ala
   1 5 10 15
<210> 26
   <211> 15
   <212> PRT
   <213> Artificial Sequence
<220>
   <223> Synthetic peptide
<400> 26
   Glu Lys Lys Tyr Ala Ala Ala Ala Ala Ala Ala Ala Ala Ala Ala
   1 5 10 15
<210> 27
   <211> 15
   <212> PRT
   <213> Artificial Sequence
<220>
   <223> Synthetic peptide
<400> 27
   Glu Ala Lys Tyr Ala Ala Ala Ala Ala Ala Ala Ala Ala Ala Ala
   1 5 10 15
<210> 28
   <211> 15
   <212> PRT
   <213> Artificial Sequence
<220>
   <223> Synthetic peptide
<400> 28
   Ala Glu Tyr Ala Lys Ala Ala Ala Ala Ala Ala Ala Ala Ala Ala
   1 5 10 15
<210> 29
   <211> 15
   <212> PRT
   <213> Artificial Sequence
<220>
   <223> Synthetic peptide
<400> 29
   Ala Glu Lys Ala Tyr Ala Ala Ala Ala Ala Ala Ala Ala Ala Ala
   1 5 10 15
<210> 30
   <211> 15
   <212> PRT
   <213> Artificial sequence
<220>
   <223> Synthetic peptide
<400> 30
   Glu Lys Tyr Ala Ala Ala Ala Ala Ala Ala Ala Ala Ala Ala Ala
   1 5 10 15
<210> 31
   <211> 15
   <212> PRT
   <213> Artificial sequence
<220>
   <223> synthetic peptide
<400> 31
   Ala Tyr Lys Ala Glu Ala Ala Ala Ala Ala Ala Ala Ala Ala Ala
   1 5 10 15
<210> 32
   <211> 15
   <212> PRT
   <213> Artificial sequence
<220>
   <223> Synthetic peptide
<400> 32
   Ala Lys Tyr Ala Glu Ala Ala Ala Ala Ala Ala Ala Ala Ala Ala
   1 5 10 15

## Description

### FIELD OF THE INVENTION

The present disclosure relates to compositions for use in treatment of psychiatric disorders and, in particular, to Copolymer 1 and related peptides and polypeptides and T cells treated therewith for use in such compositions and methods.

**Abbreviations: AMPH:** D-amphetamine sulfate; **ASR:** acoustic startle response; **BDNF:** brain-derived neurotrophic factor; **CBC:** cut-off behavioral criteria; **CFA:** complete Freund's adjuvant; **CNS:** central nervous system; **COP-1:** copolymer 1; **EPM:** Elevated plus-maze; **MBP:** myelin basic protein; **MK-801:** (+)dizocilpine maleate; **MWM:** Morris water maze; **PNS:** peripheral nervous system; **PPI:** prepulse inhibition; **PTSD:** post-traumatic stress disorder; **SCID:** severe combined immune deficiency; **Teff:** effector T cells; **Treg:** regulatory T cells; **WT:** wild-type.

### BACKGROUND

Mental disorders are now known to be characterized not only by behavioral abnormalities but also by somatic manifestations. In a number of psychiatric disorders, a neurodegenerative component has been identified. In schizophrenia, for example, there is loss of hippocampal volume and death of hippocampal neurons (Lieberman et al., 2001; Velakoulis et al., 1999), as well as anatomical and molecular abnormalities of excitatory neurons in the dorsolateral prefrontal cortex (McCullumsmith et al., 2002; Lewis et al., 1999). The etiology and pathogenesis of schizophrenia are still obscure, although there is general agreement that genetic predisposition is a significant factor (Brzustowicz et al., 2002; Falkai et al., 2003).

Symptoms of schizophrenia can be classified as positive, negative, and cognitive, by using standard rating scales such as the Positive and Negative Symptom Scale (Javitt et al., 1999). Positive symptoms include hallucinations, agitation and paranoia; negative symptoms reflect the loss of interpersonal drive and normal interest in the environment; and cognitive symptoms include conceptual disorganization and disorientation (Javitt et al., 1999). Whereas the positive symptoms usually respond well to dopamine-receptor antagonists (which however have significant and devastating side effects such as induction of Parkinson's disease), the negative symptoms and cognitive deficits typically persist, resulting in chronic morbidity and poor long-term outcome (Rummel et al., 2003).

Until quite recently, the body's principal adaptive responses to stressful stimuli were attributed to the hypothalamic-pituitary-adrenocortical axis (de Kloet, 2003). An association between the immune system and the cognitive performance, anxiety, and sensorimotor dysfunction seen in mental disorders or acute psychological stress has generally been considered unlikely or of little significance, despite a growing body of evidence suggesting that such an association not only exists, but might be an important consideration in the design of therapy (Raison et al., 2001).

Contrary to long-held belief, the effect of the immune system on the nervous system can also be beneficial. Recent studies have shown that the injured CNS can benefit from the presence of a well-controlled adaptive immunity (Schwartz et al., 1999a, 1999b; Wekerle, 2002). T cells specifically reactive to proteins that reside in the site of the insult promote post-injury neuronal survival and restoration of function (Hauben et al., 2000; Mizrahi, 2002). It was further shown that this beneficial post-injury response of autoimmune T cells to site-specific proteins is evoked spontaneously (Yoles et al., 2001; Kipnis et al., 2002).

Immune abnormalities have been reported in patients with schizophrenia, and there have been numerous attempts to find a connection between schizophrenia and autoimmune disease. However, studies over the last 60 years aimed at identifying schizophrenia as an autoimmune disease have so far been unsuccessful (Amital and Shoenfeld, 1993 and Rothermundt et al. 2001).

A link between brain maintenance and peripheral adaptive immunity was suggested by recent findings that the ability to cope with neurodegenerative conditions depends on CD4⁺T cells (Moalem et al., 1999; Kipnis et al., 2001; Yoles et al., 2001). The beneficial effect of CD4⁺ T cells in fighting off mediators of degeneration was found to be specific to antigens residing in the site of the lesion. Naturally occurring CD4⁺CD25⁺ regulatory T cells (Treg) normally suppress the ability to spontaneously evoke this neuroprotective response, which is amenable to boosting by weakening of Treg (Kipnis et al., 2002) or by a well-controlled vaccination with self-antigens or with weak agonists of self-antigens (Kipnis et al., 2000) such as Copolymer-1.

Copolymer 1, also called Cop 1, is a random non-pathogenic synthetic copolymer, a heterogeneous mix of polypeptides containing the four amino acids L-glutamic acid (E), L-alanine (A), L-tyrosine (Y) and L-lysine (K) in an approximate ratio of 1.5:4.8:1:3.6, but with no uniform sequence. Although its mode of action remains controversial, Cop 1 clearly helps retard the progression of human multiple sclerosis (MS) and of the related autoimmune condition studied in mice, experimental autoimmune encephalomyelitis (EAE, Teitelbaum et al. 1997). One form of Cop 1, known as glatiramer acetate, has been approved in several countries for the treatment of multiple sclerosis under the trademark Copaxone® (Teva Pharmaceutical Industries Ltd., Petach Tikva, Israel).

Vaccination with Cop 1 or with Cop 1-activated T cells have been shown by the present inventors to boost the protective Autoimmunity, after traumatic central nervous system (CNS) insult, thereby reducing further injury-induced damage, and can further protect CNS cells from glutamate toxicity. Reference is made to Applicant's published International Applications Nos. WO 01/52878, WO 01/93893, WO 2005/046719 and US 2002/0037848, which disclose that Cop 1, Cop 1-related peptides and polypeptides and T cells activated therewith prevent or inhibit neuronal degeneration and promote nerve regeneration in the CNS or peripheral nervous system (PNS), and protect CNS cells from glutamate toxicity.

The main inventor, Prof. Schwartz, and colleagues have shown that Cop 1 acts as a low-affinity antigen that activates a wide range of self-reacting T cells, resulting in neuroprotective autoimmunity that is effective against both CNS white matter and grey matter degeneration (Kipnis and Schwartz, 2002). The neuroprotective effect of Cop I vaccination was demonstrated by the inventors in animal models of acute and chronic neurological disorders such as optic nerve injury (Kipnis et al., 2000), head trauma (Kipnis et al., 2003), glaucoma (Schori et al., 2001), amyotrophic lateral sclerosis (Angelov et al., 2003) and in the applicant's patent applications WO 01/52878, WO 01/93893 and WO 03/047500.

Reference is made to copending international application of the same applicant entitled "Compositions and methods for treatment of psychiatric disorders", filed on the same date at the Israel Patent Office/Receiving Office, in which the present invention is explicitly excluded.

Citation of any document herein is not intended as an admission that such document is pertinent prior art, or considered material to the patentability of any claim of the present application. Any statement as to content or a date of any document is based on the information available to applicant at the time of filing and does not constitute an admission as to the correctness of such a statement.

### SUMMARY OF THE INVENTION

The present invention relates to the embodiments as defined in the claims. It has now been found, in accordance with the present disclosure, that vaccination with Copolymer 1 can lessen behavioral abnormalities and improve cognitive function in mice from dopamine imbalance induced by MK-801 or amphetamine.

In one aspect, the present invention relates to a pharmaceutical composition, preferably a vaccine, for use in treatment of psychiatric disorders, diseases or conditions which comprises a pharmaceutically acceptable carrier and an agent selected from the group consisting of (i) Copolymer 1, (ii) a Copolymer 1-related peptide, (iii) a Copolymer 1-related polypeptide, and (iv) T cells treated with (i), (ii) or (iii), wherein the psychiatric disorder, disease or condition is selected from post-traumatic stress disorder (PTSD), depression, bipolar disorder and schizophrenia.

In a further aspect, the present invention relates to the use of an agent selected from the group consisting of (i) Copolymer 1, (ii) a Copolymer 1-related peptide, (iii) a Copolymer 1-related polypeptide, and (iv) T cells treated with (i), (ii) or (iii), for the preparation of a pharmaceutical composition, preferably a vaccine, for the treatment of psychiatric disorders, diseases or conditions wherein the psychiatric disorder, disease or condition is selected from post-traumatic stress disorder (PTSD), depression, bipolar disorder and schizophrenia.

The psychiatric disorders, diseases or conditions that are treated according to the disclosure includes: (i) post-traumatic stress disorder (PTSD), (ii) depression, bipolar disorders and; (iii) schizophrenia and related disorders such as brief psychotic disorder, schizophreniform disorder, schizoaffective disorder and delusional disorder;

In the most preferred embodiment, the individual suffering from a psychiatric disorder, disease or condition is immunized with Copolymer 1.

According to the disclosure, Copolymer 1 will also treat cognitive disfunction caused by schizophrenia or by bipolar disorder.

### BRIEF DESCRIPTION OF THE FIGURES

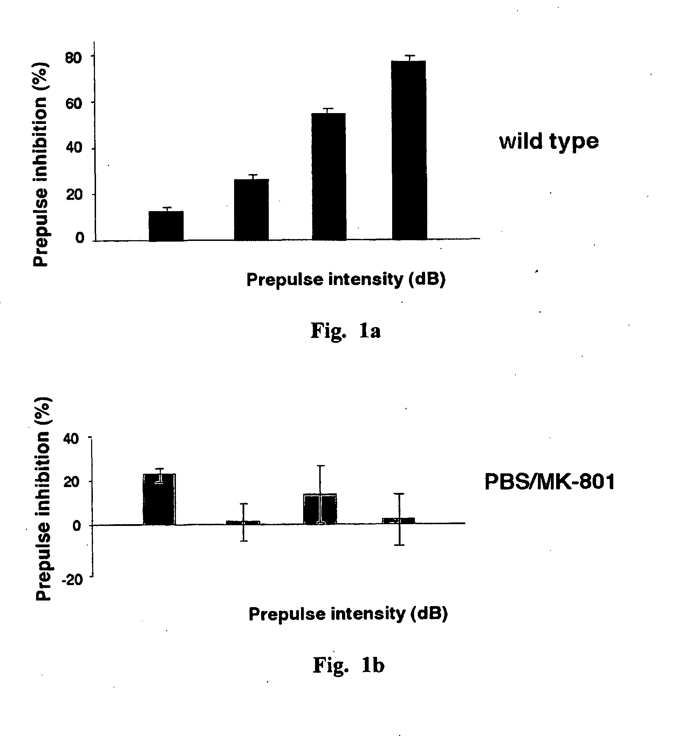

**Figs. 1a-1e** show restoration of impaired prepulse inhibition (PPI) of the acoustic startle response (ASR) in C57BL/6J mice by Cop-1 immunization. C57BL/6J mice were immunized with Cop-1/CFA or with PBS/CFA. Naive mice were used as controls. One week later, the immunized mice were injected with MK-801 (0.1 mg/kg, i.p.; 1b, 1c) or with D-amphetamine sulfate (AMPH) (2.5 mg/kg i.p.; 1d and 1c). The PPI of the ASR in control mice served as a baseline (1a). Vehicle-immunized mice injected with MK-801 showed significantly disrupted PPI (1b). In mice immunized with Cop-1/CFA, PPI was monotonically increased as a function of prepulse intensity (1c; *F* (1,9)=14.05, *P* <0.005). Vehicle-immunized mice injected with AMPH also showed significantly disrupted PPI (1d). In mice immunized with Cop-1/CFA, PPI was monotonically increased as a function of prepulse intensity (4e; *F* (1,10) = 8.6, *P* < 0.015).

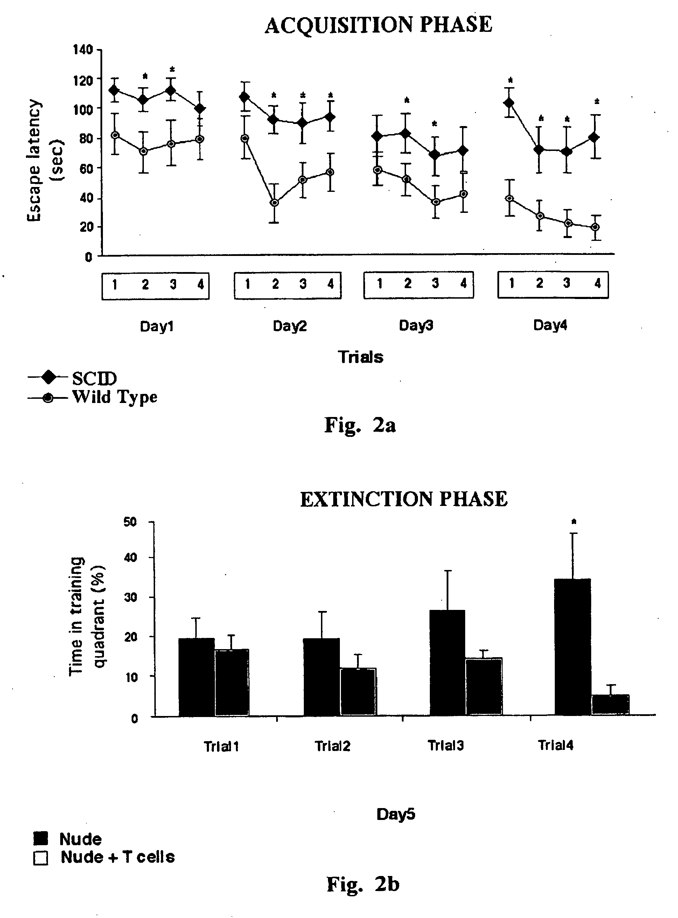

**Figs. 2a-2f** show that cognitive activity is affected by the integrity of the immune system. BALB/c/OLA wild-type (WT), nude, and SCID mice were monitored while attempting a spatial learning memory task in the Morris water maze (MWM) behavioral test. (2a-2c) Comparison of WT and SCID mice. During the acquisition (2a), extinction (2b), and reversal (2c) phases of the task, SCID mice took significantly longer than WT mice to acquire the spatial learning needed (3-way ANOVA, repeated measures: groups, df (1,20), *F* = 23.0, *P* <0.0001; trials, df (3,60), *F* = 10.995, *P* < 0.00001; days, df (1,60), *F* = 4.6, *P* < 0.006, for the acquisition phase; and groups, df (1,20), *F* = 7.9, *P* < 0.01; trials, df (3,60), *F* = 10.77, *P* < 0.00001; days, df (1,20), *F* = 34.4, *P* < 0.001, for the reversal phase). The presented results are from one of two experiments performed, with 10 mice per group in each experiment. (2d-2f) Comparison of nude mice with nude mice that were replenished with T cells 3 weeks before being tested on the MWM. During the acquisition (2d), extinction (2e), and reversal (2f) phases of the task, nonreplenished nude mice took significantly longer to acquire spatial learning than nude mice replenished with T cells from naive wild-type mice (3-way ANOVA, repeated measures: groups, df(1,18), *F* = 32.3, *P* < 0.00001; trials, df (3,54), *F* =10.1, *P* < 0.00001; days, df (3,54), *F* = 20.56, *P* < 0.00001, for the acquisition phase; and groups, df(1,18), *F* = 58.6, *P* < 0.00001; trials, df (3,54), *F* = 12.6, *P* < 0.00001; days, df(1,18), *F* = 19.2, *P* < 0.0004, for the reversal phase). The presented results are from one of two experiments performed, with 10 mice per group in each experiment.

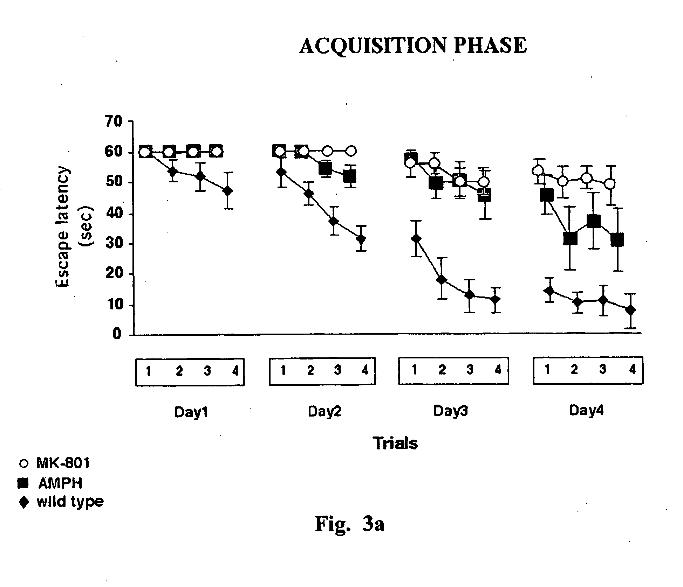

**Figs. 3a-3i** show the effect of Cop-1 vaccination on the performance by C57BL/6J mice of a spatial learning/memory task in the MWM after injection of a psychotomimetic drug. Acquisition of the spatial learning task in the MWM took significantly longer in mice injected with MK-801 (0.1 mg/kg i.p.) or AMPH (2.5 mg/kg i.p.) than in naive (PBS-injected) C57BL/6J mice in the acquisition (3a) and the reversal (3b) phases. Immunization with Cop-1/CFA resulted in decreased escape latencies in acquisition and reversal phases after injection of [MK-801 (3c, 3d) or amphetamine (3e, 3f). MK-801 (3c, 3d); 3-way ANOVA, repeated measures: groups, df(1,9), *F* =56.6, *P* < 0.0001; trials, df (3,27), *F* = 54.0, *P* < 0.00001; days, df (3,27), *F* = 15.6, *P* < 0.00001, for the acquisition phase; and groups, df(1,9), *F* = 42.7, *P* < 0.0001; trials, df (3,27), *F* = 24.4, *P* < 0.00001; days, df(1,9), *F* = 7.9, *P* < 0.02, for the reversal phase; or AMPH (3e, 3f); 3-way ANOVA, repeated measures: groups, df (1,10), *F* = 9.8, *P* < 0.01; trials, df (3,30), *F* = 29.9, *P* < 0.00001; days, df (1,30), *F* = 21.3, *P* < 0.00001, for the acquisition phase; and groups, df(1,10), *F* = 53.7, *P* < 0.00003; trials, df (3,30), *F* = 16.1, *P* < 0.00002; days, df(1,10), *F* = 5.0, *P* < 0.05 for the reversal phase]. The performance of mice immunized with Cop-1 did not differ significantly from normal behavior. (3h) The decrease in time spent in a training quadrant, obtained in control mice, was abolished upon injection of MK-801 (3g); however, mice immunized with Cop-1 behaved similarly to WT mice (3i).

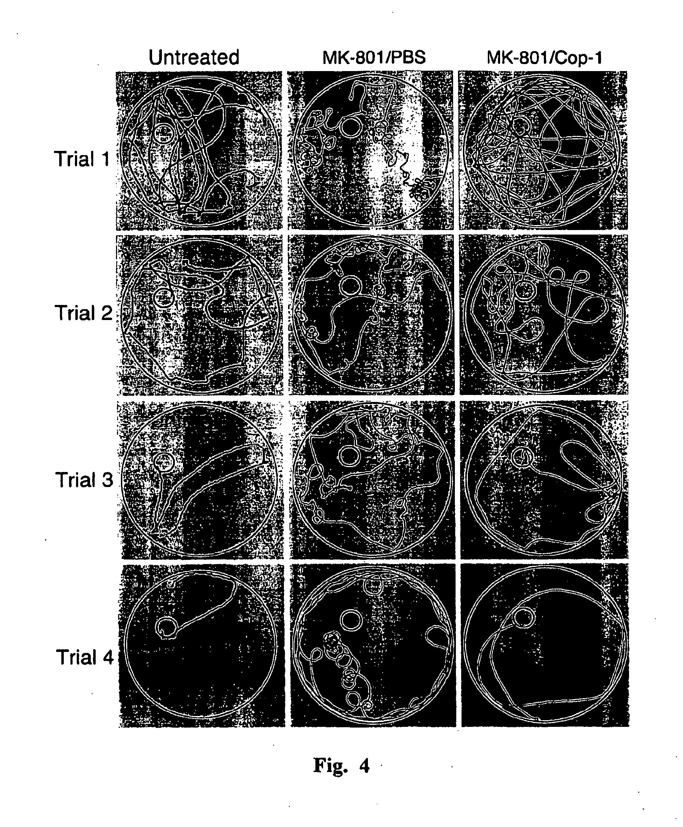

**Fig. 4** illustrates tracking of Cop-1-immunized and control mice in the Morris water maze after injection of MK-801 and shows that Cop-1 counteracted MK-801-induced impairment of learning and memory. The figure depicts the swimming strategies of C57BL/6J mice that were immunized with Cop-1/CFA or PBS/CFA, and injected 1 week later with MK-801. Naive mice served as controls for performance in the MWM. On the second day after administration of the drug, performance was tested in four consecutive trials at 5-min intervals. The Cop-1-vaccinated mice, like the naïve mice, learned to swim away from the wall to search for the platform, in the inner 50% of the pool and to use the platform as a refuge when they found it. A significantly less efficient strategy was used by MK-801-injected mice immunized with vehicle.

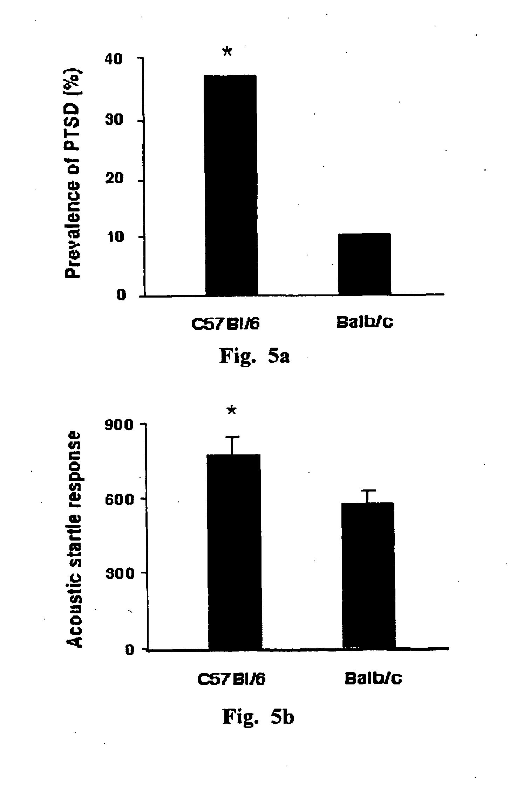

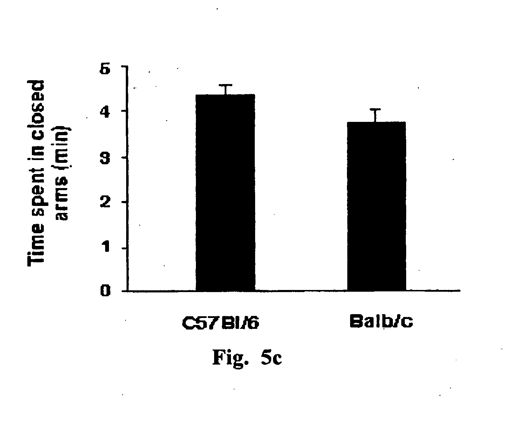

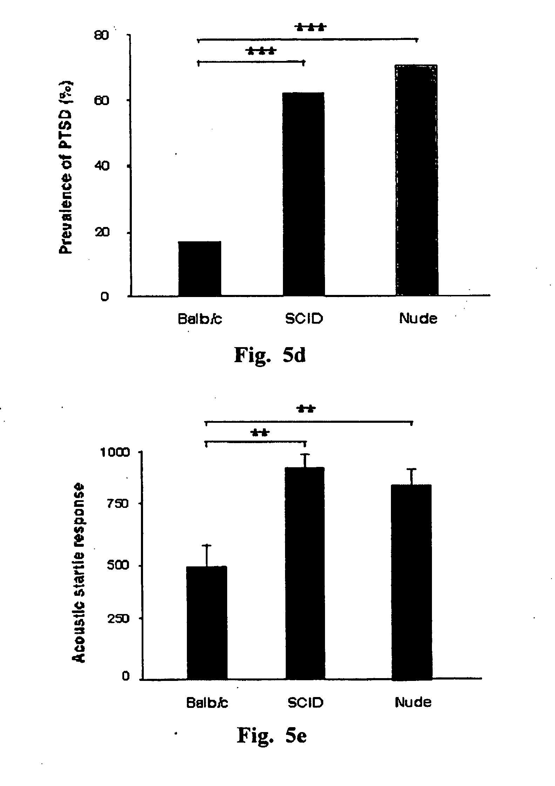

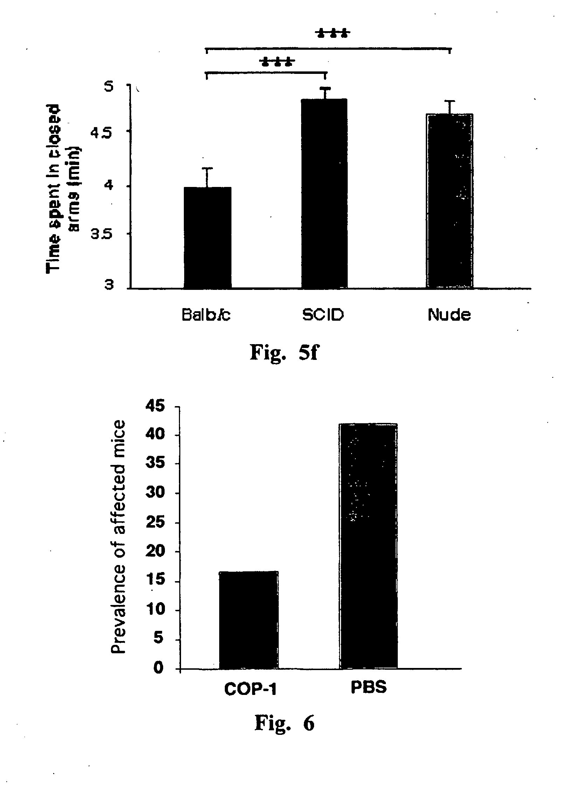

**Figs. 5a-5f** show strain dependence and T-cell dependence on the ability to withstand psychological stress. (Figs. 5a, 5d) Mouse strains differ in their ability to adapt to psychological stress. A single 10-min exposure to the odor of a predator caused behavioral changes in 36.8% of male C57BL/6J mice but in only 10.5% of male BALB/c mice (χ2 =3.7, P<0.05). (Figs. 5b, 5e) In the BALB/c strain, maladaptation was significantly more prevalent in SCID mice (61.9%) than in the wild type (17.2%; χ2 =10.6, P<0.001). (Figs. 5c, 5f). Maladaptation was similarly more prevalent in nude mice (devoid of mature T cells only) than in the wild type (70% and 17.2%, respectively; χ2 test: P<0.0002), verifying that the observed differences were attributable to the absence of mature T cells.

**Fig. 6** shows the ability of mice immunized with Cop-1 to withstand psychological stress in comparison with PBS-treated mice (control), after a single 10-min exposure to the odor of a predator.

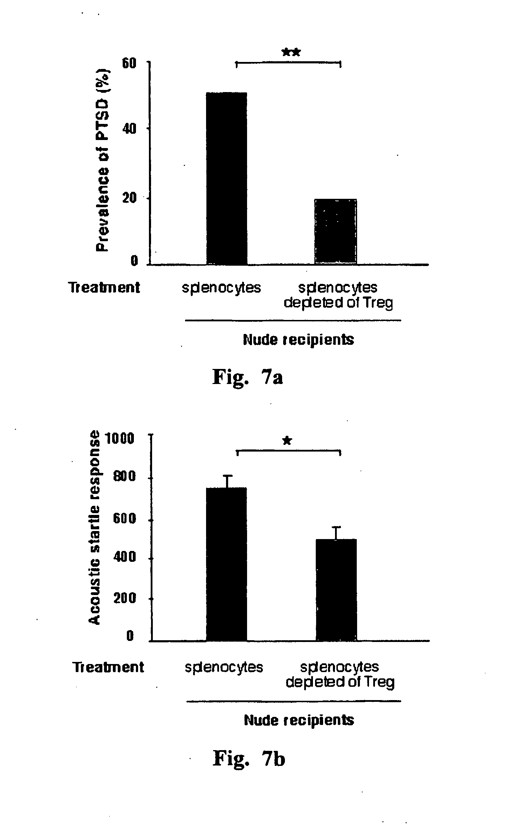

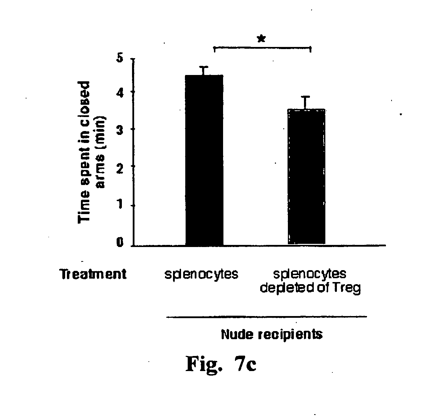

**Figs. 7a-7c** show that naturally occurring CD4+CD25+ regulatory T cells suppress the ability to withstand psychological stress. (Fig. 7a) A single 10-min exposure to the odor of a predator resulted in maladaptation in 70% of nude male BALB/c mice (see Fig. 6). The prevalence of maladaptation was somewhat decreased (50%) in nude mice that were replenished with normal splenocytes from wild-type BALB/c mice. In nude mice that were replenished with a splenocyte population depleted of Treg, the prevalence of maladaptation (20%) was significantly lower than that of control (non-replenished) nude mice (χ2=6.7, P<0.009). (Fig. 7b) The startle response (mean ± SD) of nude mice replenished with splenocytes depleted of Treg was significantly weaker than that of nude mice replenished with a normal splenocyte population (P<0.03) or than that of control nude mice (F(df=2,37)=9.2, P<0.0006). (Fig. 7c) Nude mice replenished with splenocytes depleted of Treg spent significantly less time exploring the closed arms of the elevated plus-maze than did nude mice replenished with a normal splenocyte population (P<0.02) or than control nude mice (F(df=2,37)=8.7, P<0.0008).

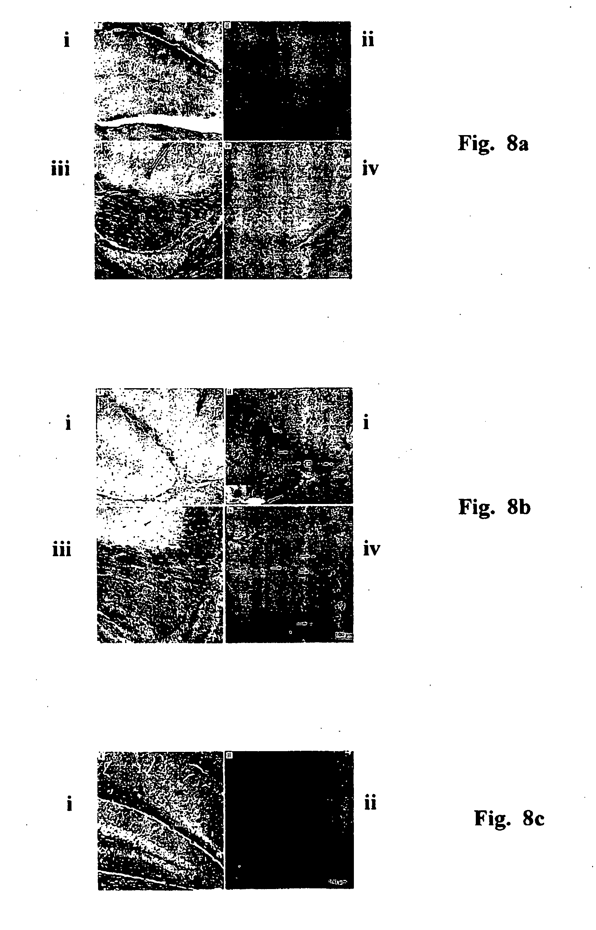

**Figs. 8a-8c** are micrographs showing that the immunohistochemistry of T cells in the brain is correlated with adaptation to psychological stress. Maladapted animals from the group of nude mice replenished with a normal splenocyte population from wild-type mice and well-adapted animals from the group of nude mice replenished with splenocytes from wild-type mice depleted of Treg were killed and their brains were removed, perfused, embedded in paraffin, and sliced for histology. Brain slices from the hippocampal area and fimbria of the hippocampus were stained for myelinated axons with Luxol and counterstained with eosin, or stained with anti-CD3 antibodies for the presence of T cells and counterstained with hematoxylin. (Fig. 8a) Brain slices from the maladapted mice showed no staining for T cells (ii and iv). (Fig. 8b) Brain slices from the well-adapted mice showed T-cell reactivity in hippocampal areas (ii and iv) corresponding to myelin reactivity (Luxol-positive areas; i and iii). (Fig. 8c) Wild-type mice that were not exposed to stress showed, as expected, no T-cell reactivity in brain slices. The micrographs show representative results of at least 6 brain slices from each mouse, and from at least 3 mice in each group.

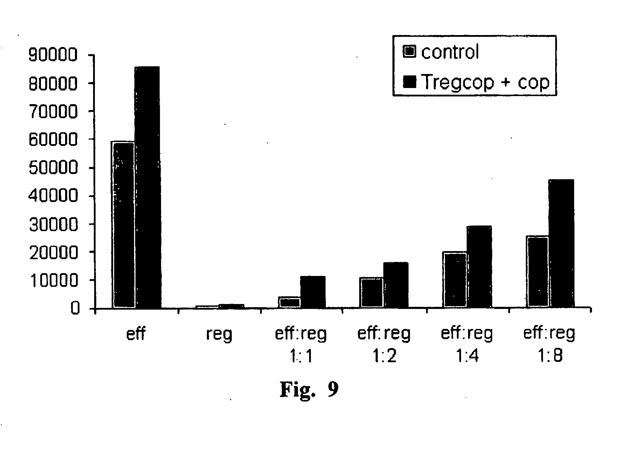

**Fig. 9** shows that Cop-1 alleviates the suppressive activity mediated by Treg (CD4⁺CD25⁺). T cell proliferation was assayed by incorporation of [³H]-thymidine into effector T cells (Teff) co-cultured with Treg. Recorded values are from one representative experiment out of three and are expressed as means ± SD of 4 replicates.

### DETAILED DESCRIPTION OF THE INVENTION

The present invention relates to treatment of a psychiatric disorder, disease or condition which comprises administering to an individual in need of such a treatment an effective amount of an agent selected from the group consisting of (i) Copolymer 1, (ii) a Copolymer 1-related peptide, (iii) a Copolymer 1-related polypeptide, and (iv) T cells treated with (i), (ii) or (iii) wherein the psychiatric disorder, disease or condition is selected from post-traumatic stress disorder (PTSD), depression, bipolar disorder and schizophrenia.

We examined whether adaptive immunity plays a role in higher brain functions, both under normal conditions and in the abnormal situation generated by neurotransmitter imbalance. It is shown herein that systemic immune deficiency resulted in cognitive impairment which could be reversed by replenishment with T cells. Moreover, vaccination with Cop-1, a synthetic lowaffinity agonist of a wide-range of self-reactive T cell clones, presumably by boosting the number of T cells capable of crossreacting with relevant CNS antigens, prevented drug-induced psychosis and reduced cognitive impaiment.

The results herein also show that another brain function critically affected by the integrity of the peripheral adaptive immune system is cognition. When tested in the MWM (on a task that generates stress), immune-deficient mice showed impaired cognitive function, which was prevented by restoration of immune system integrity. The observed impairment of cognitive activity, which occurred here when the immune system was intact but the brain was suffering from an imbalance in neurotransmitters, suggests that the peripheral immune system can contain the small fluctuations in neurotransmitter levels that occur daily and enable normal cognitive performance, but is unable to cope with a pathological imbalance that causes cognitive impairment. In the latter situation, therefore, the relevant T cells need to be boosted. Boosting was achieved by vaccination with Cop-1, which significantly prevented the cognitive impairment induced by psychotomimetic drugs. Both behavioral and cognitive abnormalities were observed in our mouse model within 15 min of administration of MK-801 or AMPA and were counteracted by the effects of the Cop-1 vaccination given 1 week earlier. Due to the rapid onset of symptoms that occurs in the experimental paradigm used here, the mice had to be vaccinated before psychosis was induced. It should be noted, however, that the vaccination according to the present disclosure encompasses not only preventive use, but also as a remedial therapy for chronic patients, in whom intervention at any stage of the disease should be beneficial.

The rapidity of the Cop-1-reactive T cells in counteracting the psychotic effects of the drugs suggests that T cells already elicited by the immunization were patrolling the healthy brain. It was recently shown that Cop-1 vaccination in healthy animals indeed leads to increased accumulation of T cells in the CNS and local production of BDNF (Kipnis et al., 2000). It is also possible that, as a result of the early immunization, a significant number of Cop-1-reactive T cells circulate in the blood, and that they home to the relevant site only when it is under stress, caused for example by a pathological alteration in the brain level of dopamine (by injection of AMPH) or of glutamate (by injection of MK-801). Regardless of whether the effector T cells circulate in the blood or reside in the brain, the rapidity of the T cell response to neurotransmitter imbalance testifies to the importance of well functioning adaptive immunity in the daily maintenance of the brain (Kipnis and Schwartz, 2002). These results might also explain why, in the elderly population, who are known to suffer a disproportionate reduction in immunity and increased metabolic and neurotransmitter imbalance in the brain, the incidence of dementia is increased (Wick et al., 2003).

Psychological trauma, like physical insults to the CNS, can cause widespread, long-term changes in neurological and neurohormonal functioning, which seem to be related to morphological changes (Markowitsch et al., 1998). It seems reasonable to assume that exacerbation of both the behavioral and the cognitive manifestations of schizophrenia over time can be correlated with the neurodegeneration occurring in certain regions of the brain (Deutsch et al., 2001). The present finding that Cop-1-reactive T cells in mice mediated the prevention of MK-801-induced or amphetamine-induced psychoses, which mimic in part symptoms of schizophrenia in humans, coupled with the increasing recognition that neurodegeneration plays a role in schizophrenia and other mental diseases, suggests that the development of immune-based neuroprotection might provide a global remedy that addresses both positive and negative symptoms of schizophrenia and possibly also of other psychiatric conditions, including age-related and HIV-related dementias.

It is shown herein that a cross-talk between the brain and the adaptive immune system (T cells) affects the consequences of a single instance of psychological trauma. Complete T cell deficiency was found here to correlate with maladaptation to psychological stress, whereas removal of only a subpopulation of T cells, the naturally occurring regulatory T cells (Treg), improved the ability to adapt to the stress. This indicates that in normal animals subjected to traumatic mental stress, the T cell-mediated response cannot reach its full therapeutic potential, as it is suppressed by the presence of the naturally occurring regulatory T cells. Down-regulation of Treg by Cop-1, as shown herein, is beneficial and can improve an individual's ability to withstand and cope with stressful conditions.

Our present results indeed show that boosting of a T cell response to the self-like antigen Cop-1 significantly reduced cognitive impairment and eased psychosis in animal models with schizophrenia-associated symptoms. Both behavioral and cognitive abnormalities were observed in our mouse model within 15 minutes of administration of the psychotomimetic drugs MK-801 and AMPH, and were counteracted by the effects of the Cop-1 vaccination given one week earlier. The rapidity of the Cop-1-reactive T cells in counteracting the psychotic effects of the drugs suggests that T cells had already been elicited by the immunization, and were patrolling the healthy brain in a non-activated state, though on alert and ready for action if needed. Alternatively, it is possible that as a result of the immunization a significant number of Cop-1-reactive T cells, as yet unactivated, were circulating in the blood, ready to home to the site of the threat when needed there. A pathological alteration in the brain level of dopamine (by injection of amphetamine) or of glutamate (by injection of MK-801) might activate these T cells, allowing rapid protection against the devastating effects of the neurotransmitter imbalance.

The observed effect of Cop-1 on the alleviation of psychosis induced by psychomimetic drugs indicates that it is a suitable candidate of choice for the development of a new generation of anti-psychotic drugs.

Glutamatergic imbalance is a feature common to neurodegenerative and mental disorders. After injury to the CNS, T cells directed to CNS-related self-antigens participate in CNS maintenance, which includes protection against glutamate toxicity. This neuroprotective effect can be boosted by agonists of self-antigens, such as Cop-1. As disclosed herein, it is shown that a similar T cell-dependent mechanism is protective against neurotransmitter imbalance in the brain, leading to behavioral and cognitive malfunction. Vaccination with Cop-1 protected mice from psychotic behavior and cognitive impairment induced by MK-801 or amphetamine (simulating symptoms of schizophrenia). It is also shown herein that Cop-1-reactive T cells, upon encountering relevant self-antigens (MBP), produced brain-derived neurotrophic factor (BDNF), a neurotrophin known to be protective in schizophrenic brain, to be linked to bipolar disorder development and connected with nerve activity, memory function and mood.

As used herein, the terms "Cop-1" and "Copolymer 1" are used interchangeably. For the purpose of the present invention, "Copolymer 1-related peptide or Copolymer 1-related polypeptide" is intended to include any peptide or polypeptide, including a random copolymer that cross-reacts functionally with myelin basic protein (MBP) and is able to compete with MBP on the MHC class II in the antigen presentation.

The composition or vaccine disclosed herein may comprise as active agent a random copolymer comprising a suitable quantity of a positively charged amino acid such as lysine or arginine, in combination with a negatively charged amino acid (preferably in a lesser quantity) such as glutamic acid or aspartic acid, optionally in combination with a non-charged neutral amino acid such as alanine or glycine, serving as a filler, and optionally with an amino acid adapted to confer on the copolymer immunogenic properties, such as an aromatic amino acid like tyrosine or tryptophan. Such compositions may include any of those copolymers disclosed in WO 00/05250.

More specifically, the composition disclosed herein comprises at least one copolymer selected from the group consisting of random copolymers comprising one amino acid selected from each of at least three of the following groups: (a) lysine and arginine; (b) glutamic acid and aspartic acid; (c) alanine and glycine; and (d) tyrosine and tryptophan.

The copolymers disclosed herein can be composed of L- or D-amino acids or mixtures thereof. As is known by those of skill in the art, L-amino acids occur in most natural proteins. However, D-amino acids are commercially available and can be substituted for some or all of the amino acids used to make the terpolymers and other copolymers disclosed herein. The present' disclosure contemplates the use of copolymers containing both D- and L-amino acids, as well as copolymers consisting essentially of either L- or D-amino acids.

In a more preferred embodiment, a pharmaceutical composition or vaccine disclosed herein comprises Copolymer 1, a mixture of random polypeptides consisting essentially of the amino acids L-glutamic acid (E), L-alanine (A), L-tyrosine (Y) and L-lysine (K) in an approximate ratio of 1.5:4.8:1:3.6, having a net overall positive electrical charge and of a molecular weight from about 2 KDa to about 40 KDa. In one preferred embodiment, the Cop 1 has average molecular weight of about 2 KDa to about 20 KDa, more preferably of about 4,7 KDa to about 13 K Da, still more preferably of about 4 KDa to about 8.6 KDa, of about 5 KDa to 9 KDa, or of about 6.25 KDa to 8.4 KDa. In another preferred embodiment, the Cop 1 has average molecular weight of about 13 KDa to about 20 KDa, more preferably of about 13 KDa to about 16 KDa or of about 15 KDa to about 16 KDa. Other average molecular weights for Cop 1, lower than 40 KDa, are also encompassed by the present disclosure. Copolymer 1 of said molecular weight ranges can be prepared by methods known in the art, for example by the processes described in U.S. Patent No. 5,800,808.

The Copolymer 1 may be a polypeptide comprising from about 15 to about 100, preferably from about 40 to about 80, amino acids in length. In one preferred embodiment, the Cop 1 is in the form of its acetate salt known under the generic name glatiramer acetate, that has been approved in several countries for the treatment of multiple sclerosis (MS) under the trade name, Copaxone® (a trademark of Teva Pharmaceuticals Ltd., Petach Tikva, Israel). The activity of Copolymer 1 for the vaccine disclosed herein is expected to remain if one or more of the following substitutions is made: aspartic acid for glutamic acid, glycine for alanine, arginine for lysine, and tryptophan for tyrosine.

In another embodiment, the Cop 1-related peptide or polypeptide is a copolymer of three different amino acids each from a different one of three groups of the groups (a) to (d). These copolymers are herein referred to as terpolymers.

In one embodiment, the Cop 1-related peptide or polypeptide is a terpolymer containing tyrosine, alanine, and lysine, hereinafter designated YAK, in which the average molar fraction of the amino acids can vary: tyrosine can be present in a mole fraction of about 0.05-0.250; alanine in a mole fraction of about 0.3 - 0.6; and lysine in a mole fraction of about 0.1-0.5. More preferably, the molar ratios of tyrosine, alanine and lysine are about 0.10:0.54:0.35, respectively. It is possible to substitute arginine for lysine, glycine for alanine, and/or tryptophan for tyrosine.

In another embodiment, the Cop 1-related peptide or polypeptide is a terpolymer containing tyrosine, glutamic acid, and lysine, hereinafter designated YEK, in which the average molar fraction of the amino acids can vary: glutamic acid can be present in a mole fraction of about 0.005 - 0.300, tyrosine can be present in a mole fraction of about 0.005-0.250, and lysine can be present in a mole fraction of about 0.3-0.7. More preferably, the molar ratios of glutamic acid, tyrosine, and lysine are about 0.26:0.16:0.58, respectively. It is possible to substitute aspartic acid for glutamic acid, arginine for lysine, and/or tryptophan for tyrosine.

In another preferred embodiment, the Cop 1-related peptide or polypeptide is a terpolymer containing lysine, glutamic acid, and alanine, hereinafter designated KEA, in which the average molar fraction of the amino acids can vary: glutamic acid can be present in a mole fraction of about 0.005-0.300, alanine in a mole fraction of about 0.005-0.600, and lysine can be present in a mole fraction of about 0.2 - 0.7. More preferably, the molar ratios of glutamic acid, alanine and lysine are about 0.15:0.48:0.36, respectively. It is possible to substitute aspartic acid for glutamic acid, glycine for alanine, and/or arginine for lysine.

In a preferred embodiment, the Cop 1-related peptide or polypeptide is a terpolymer containing tyrosine, glutamic acid, and alanine, hereinafter designated YEA, in which the average molar fraction of the amino acids can vary: tyrosine can be present in a mole fraction of about 0.005-0.250, glutamic acid in a mole fraction of about 0.005-0.300, and alanine in a mole fraction of about 0.005-0.800. More preferably, the molar ratios of glutamic acid, alanine, and tyrosine are about 0.21: 0.65:0.14, respectively. It is possible to substitute tryptophan for tyrosine, aspartic acid for glutamic acid, and/or glycine for alanine.

The average molecular weight of the terpolymers YAK, YEK, KEA and YEA can vary between about 2 KDa to 40 KDa, preferably between about 3 KDa to 35 KDa, more preferably between about 5 KDa to 25 KDa.

Copolymer 1 and related peptides and polypeptides may be prepared by methods known in the art, for example, under condensation conditions using the desired molar ratio of amino acids in solution, or by solid phase synthetic procedures. Condensation conditions include the proper temperature, pH, and solvent conditions for condensing the carboxyl group of one amino acid with the amino group of another amino acid to form a peptide bond. Condensing agents, for example dicyclohexylcarbodiimide, can be used to facilitate the formation of the peptide bond. Blocking groups can be used to protect functional groups, such as the side chain moieties and some of the amino or carboxyl groups against undesired side reactions.

For example, the copolymers can be prepared by the process disclosed in U.S. Patent 3,849,550, wherein the N-carboxyanhydrides of tyrosine, alanine, γ-benzyl glutamate and N ε-trifluoroacetyl-lysine are polymerized at ambient temperatures (20°C-26°C) in anhydrous dioxane with diethylamine as an initiator. The γ-carboxyl group of the glutamic acid can be deblocked by hydrogen bromide in glacial acetic acid. The trifluoroacetyl groups are removed from lysine by 1M piperidine. One of skill in the art readily understands that the process can be adjusted to make peptides and polypeptides containing the desired amino acids, that is, three of the four amino acids in Copolymer 1, by selectively eliminating the reactions that relate to any one of glutamic acid, alanine, tyrosine, or lysine.

The molecular weight of the copolymers can be adjusted during polypeptide synthesis or after the copolymers have been made. To adjust the molecular weight during polypeptide synthesis, the synthetic conditions or the amounts of amino acids are adjusted so that synthesis stops when the polypeptide reaches the approximate length which is desired. After synthesis, polypeptides with the desired molecular weight can be obtained by any available size selection procedure, such as chromatography of the polypeptides on a molecular weight sizing column or gel, and collection of the molecular weight ranges desired. The copolymers can also be partially hydrolyzed to remove high molecular weight species, for example, by acid or enzymatic hydrolysis, and then purified to remove the acid or enzymes.

In one embodiment, the copolymers with a desired molecular weight may be prepared by a process, which includes reacting a protected polypeptide with hydrobromic acid to form a trifluoroacetyl-polypeptide having the desired molecular weight profile. The reaction is performed for a time and at a temperature which is predetermined by one or more test reactions. During the test reaction, the time and temperature are varied and the molecular weight range of a given batch of test polypeptides is determined. The test conditions which provide the optimal molecular weight range for that batch of polypeptides are used for the batch. Thus, a trifluoroacetyl-polypeptide having the desired molecular weight profile can be produced by a process, which includes reacting the protected polypeptide with hydrobromic acid for a time and at a temperature predetermined by test reaction. The trifluoroacetyl-polypeptide with the desired molecular weight profile is then further treated with an aqueous piperidine solution to form a low toxicity polypeptide having the desired molecular weight.

In a preferred embodiment, a test sample of protected polypeptide from a given batch is reacted with hydrobromic acid for about 10-50 hours at a temperature of about 20-28°C. The best conditions for that batch are determined by running several test reactions. For example, in one embodiment, the protected polypeptide is reacted with hydrobromic acid for about 17 hours at a temperature of about 26°C.

As binding motifs of Cop 1 to MS-associated HLA-DR molecules are known (Fridkis-Hareli et al, 1999), polypeptides derived from Cop 1 having a defined sequence can readily be prepared and tested for binding to the peptide binding groove of the HLA-DR molecules as described in the Fridkis-Hareli et al (1999) publication. Examples of such peptides are those disclosed in WO 00/05249 and WO 00/05250 include the peptides of SEQ ID NOs. 1-32 hereinbelow.

**Table 1**

| **SEQ ID NO.** | **Peptide Sequence** |
|---|---|
| **1** | AAAYAAAAAAKAAAA |
| **2** | AEKYAAAAAAKAAAA |
| **3** | AKEYAAAAAAKAAAA |
| **4** | AKKYAAAAAAKAAAA |
| **5** | AEAYAAAAAAKAAAA |
| **6** | KEAYAAAAAAKAAAA |
| 7 | AEEYAAAAAAKAAAA |
| 8 | AAEYAAAAAAKAAAA |
| 9 | EKAYAAAAAAKAAAA |
| 10 | AAKYEAAAAAKAAAA |
| 11 | AAKYAEAAAAKAAAA |
| 12 | EAAYAAAAAAKAAAA |
| 13 | EKKYAAAAAAKAAAA |
| 14 | EAKYAAAAAAKAAAA |
| 15 | AEKYAAAAAAAAAAA |
| 16 | AKEYAAAAAAAAAAA |
| 17 | AKKYEAAAAAAAAAA |
| 18 | AKKYAEAAAAAAAAA |
| 19 | AEAYKAAAAAAAAAA |
| 20 | KEAYAAAAAAAAAAA |
| 21 | AEEYKAAAAAAAAAA |
| 22 | AAEYKAAAAAAAAAA |
| 23 | EKAYAAAAAAAAAAA |
| 24 | AAKYEAAAAAAAAAA |
| 25 | AAKYAEAAAAAAAAA |
| 26 | EKKYAAAAAAAAAAA |
| 27 | EAKYAAAAAAAAAAA |
| 28 | AEYAKAAAAAAAAAA |
| 29 | AEKAYAAAAAAAAAA |
| 30 | EKYAAAAAAAAAAAA |
| 31 | AYKAEAAAAAAAAAA |
| 32 | AKYAEAAAAAAAAAA |

Such peptides and other similar peptides derived from Cop 1 would be expected to have similar activity as Cop 1. Such peptides, and other similar peptides, are also considered to be within the definition of Cop 1-related peptides or polypeptides and their use is considered to be part of the present invention.

The definition of "Cop 1-related peptide or polypeptide" according to the invention is meant to encompass other synthetic amino acid copolymers such as the random four-amino acid copolymers described by Fridkis-Hareli et al., 2002 (as candidates for treatment of multiple sclerosis), namely copolymers (14-, 35- and 50-mers) containing the amino acids phenylalanine, glutamic acid, alanine and lysine (poly FEAK), or tyrosine, phenylalanine, alanine and lysine (poly YFAK), and any other similar copolymer to be discovered that can be considered a universal antigen similar to Cop 1.

In another embodiment, the present invention relates to the treatment of a psychiatric disease, disorder or condition which comprises administering to an individual in need T cells that have been activated preferably in the presence of Cop 1, or by a Cop 1-related peptide or polypeptide wherein the psychiatric disorder, disease or condition is selected from post-traumatic stress disorder (PTSD), depression, bipolar disorder and schizophrenia. Such T cells are preferably autologous, most preferably of the CD4 and/or CD8 phenotypes, but they may also be allogeneic T cells from related donors, e.g., siblings, parents, children, or HLA-matched or partially matched, semi-allogeneic or fully allogeneic donors. T cells for this purpose are described in USSN 09/756,301 and USSN 09/765,644, corresponding to WO 01/93893.

The dosage of Cop 1 to be administered will be determined by the physician according to the age of the patient and stage of the disease and may be chosen from a range of 1-80 mg, preferably 20 mg, although any other suitable dosage is encompassed by the invention. The treatment should be preferably carried out by administration of repeated doses at suitable time intervals, preferably every 1, 4 or 6 weeks, but any other suitable interval between the immunizations is envisaged according to the psychiatric disease to be treated, the age and condition of the patient.

Pharmaceutical compositions as disclosed herein may be formulated in conventional manner using one or more physiologically acceptable carriers or excipients. The carrier(s) must be "acceptable" in the sense of being compatible with the other ingredients of the composition and not deleterious to the recipient thereof.

As disclosed herein, the composition comprising Copolymer 1 or a Copolymer 1-related peptide or polypeptide is administered in a regimen that confers protective autoimmunity (also sometimes referred to as a vaccine for neuroprotective vaccination). Such a vaccine, if desired, may contain Copolymer 1 emulsified in an adjuvant suitable for human clinical use.

Thus, according to the present agent, the active agent may be administered without any adjuvant or it may be emulsified in an adjuvant suitable for human clinical use. The adjuvant is selected from aluminum hydroxide, aluminum hydroxide gel, and aluminum hydroxyphosphate, or any other adjuvant that is found to be suitable for human clinical use. In a preferred embodiment, the vaccine adjuvant is amorphous aluminum hydroxyphosphate having an acidic isoelectric point and an Al:P ratio of 1:1 (herein referred to as Alum-phos). It is clear that this is given by way of example only, and that the vaccine can be varied both with respect to the constituents and relative proportions of the constituents.

Methods of administration include, but are not limited to, parenteral, e.g., intravenous, intraperitoneal, intramuscular subcutaneous mucosal (e.g., oral, intranasal, buccal, vaginal, rectal, intraocular), intrathecal, topical and intradermal routes. Administration can be systemic or local.

As disclosed herein, Cop 1 or a Cop 1-related peptide or polypeptide may be used as a sole therapy or in combination with one or more drugs for the treatment of the psychiatric disorder, disease or condition. When administered together with another drug or drugs suitable for treatment of the psychiatric disorder, disease or condition, the additional drug or drugs is/are administered at the same day of vaccination, and daily or at any other interval thereafter, according to the manufacturer's instructions, with no association to the vaccine regimen.

The invention will now be illustrated by the following non-limiting examples.

### EXAMPLES

### Materials and Methods

***(i) Animals.*** Inbred adult male wild-type and *nu*/*nu* BALB/c and C57B1/6J mice, adult female Lewis rats and BALB/c/OLA mice, as well as BALB/c/OLA mice with severe combined immune deficiency (SCID) (due to RAG 1/2 knockout) and nude mice (deficient in mature T cells), all 8-12 weeks old, were supplied by the Animal Breeding Center of The Weizmann Institute of Science (Rehovot, Israel). The animals were housed in a light- and temperature-controlled room and matched for age in each experiment. Animals were handled according to the regulations formulated by IACUC (Institutional Animal Care and Use Committee).
***(ii) Antigens.*** Copolymer 1 (Cop-1) was purchased from Teva Pharmaceuticals Ltd. (Petach Tikva, Israel).
***(iii) Immunization.*** Each animal was injected with a total of 100 µg of Cop-1 emulsified in an equal volume of complete Freund's adjuvant (CFA) containing 5 mg/ml of Mycobacteria H37 RA (Difco). The emulsion, in a total volume of 0.1 mL, was injected into the flank 1 week before the mouse was first injected with a psychotomimetic drug. Control mice were injected with an equal volume of phosphate-buffered saline (PBS) emulsified in CFA.
***(iv) T-cell lines.*** Ten days after Cop-1 immunization, T-cell lines were generated from draining lymph node cells obtained from Lewis rats immunized with Cop-1 emulsified in CFA. The lymph nodes were surgically removed, dissociated, washed, and then activated with the antigen (10 µg/ml) in stimulation medium as previously reported (Kipnis et al., 2000). The T-cell lines were expanded by repeated stimulation and propagation.
***(v) Enzyme-linked immunosorbent assay.*** T cell-reactive Cop-1 cells were grown for 1 week in a propagation medium, then washed with PBS and resuspended in stimulation medium. The cultured T cells (0.5 x 10⁶ cells/mL) were then incubated, in the presence of irradiated thymocytes (10⁷ cells/mL), with concanavalin A (Con A; 1.25 µg/mL), myelin basic protein (MBP; 10 µg/ml), Cop-1 (10 µg/mL), ovalbumin (OVA; 10 µg/mL), or no antigen, in stimulation medium. After 48 h the cells were centrifuged and their supernatants were collected and sampled. Concentrations of brain-derived neurotrophic factor (BDNF) in the samples were determined with a sensitive sandwich ELISA. In brief, 96-well flat-bottomed plates were coated with a chicken anti-human BDNF antibody (Promega, Madison, WI) in 0.025 M NaHCO₃ and 0.025 M Na₂CO₃ (pH 8.2). Recombinant human BDNF (used as a standard; Research Diagnostics, Flanders, NJ) was used in serial dilutions in blocking solution containing 3% bovine serum albumin, 0.05% polyoxyethylene-sorbitan monolaurate (Tween-20), and 1% fetal calf serum in PBS (pH 8.2). Bound BDNF was detected by incubating the plates with a mouse anti-human BDNF antibody (Research Diagnostics) and then with peroxidase-conjugated goat anti-mouse IgG (Jackson ImmunoResearch, West Grove, PA) in blocking solution. The plates were developed using a 3,3',5,5'-tetramethylbenzidine liquid substrate system (Sigma-Aldrich). The reaction was stopped by adding 1M H₃PO₄, and the optical density was determined at 450 nm. Results for each experiment were calculated as the amount of secreted BDNF per 1 mL of sample, after subtraction of background levels of the irradiated thymocytes incubated with the stimulation medium.
***(vi) Drug solutions.*** Fresh solutions of dizocilpine maleate (MK-801; Sigma-Aldrich) were prepared in physiological saline (0.9% NaCl in sterile distilled water) for each batch of mice. Physiological saline was also used as a vehicle for D-amphetamine sulfate (AMPH; Sigma). MK-801 (0.1 mg/kg) or AMPH (2.5 mg/kg) or saline was injected i.p. in a total volume of 5 ml per kg of body weight. Mice were injected with MK-801, AMPH, or vehicle 15 min before being subjected to behavioral tests.
***(vii) Morris water maze (MWM) behavioral test.*** Spatial learning/memory was assessed by performance on a hippocampal-dependent visuospatial learning task in the MWM. Mice were given four trials per day, for four consecutive days, to find a hidden platform located 1.5 cm below the water surface in a pool 1.4 m in diameter. Within the testing room only distal visuo-spatial cues were available to the mice for location of the submerged platform. The escape latency, i.e., the time required by the mouse to find and climb onto the platform, was recorded for up to 60 s. Each mouse was allowed to remain on the platform for 30 s, and was then moved from the maze to its home cage. If the mouse did not find the platform within 120 s, it was manually placed on the platform and returned to its home cage after 30 s. The inter-trial interval was 30 s. On day 5, the platform was removed from the pool, and each mouse was tested by a probe trial for 60 s. On days 6-7 the platform was placed at the opposite location, and the mouse was retrained in four sessions. Data were recorded by using an EthoVision automated tracking system (Noldus Information Technology, Wageningen, The Netherlands).
***(viii) Prepulse inhibition (PPI).*** All sessions for testing of PPI consisted of startle trials (pulse-alone), prepulse trials (prepulse plus pulse), and no-stimulus trials (no-stim). The pulse-alone trial consisted of a 40-ms, 120-dB pulse of broadband noise. Acoustic PPI was measured by prepulse plus pulse trials consisting of a 20-ms prepulse, 100 ms delay, and then a 40-ms, 120-dB startle pulse. The onset-to-onset interval was 120 ms. Acoustic prepulse intensities were 4, 8, 13, and 16 dB above the 65-dB background noise (i.e., 69, 73, 78, and 81 dB). The no-stim trial consisted of background noise only. The acoustic section of the test session began and ended with five presentations of the pulse-alone trial; in between, each acoustic or no-stim trial type was presented 10 times in a pseudorandom order. The average time between trials was 15 s (range: 12-30 s). After the mice were placed in the startle chambers, a 65-dB background noise was presented for a 5-min period of acclimation, and then throughout the test session.
   PPI was calculated as a percentage score for each acoustic prepulse trial type: % PPI = 100 - {[(startle response for prepulse + pulse)/(startle response for pulse-alone)] x 100}. The magnitude of the acoustic startle response was calculated as the average response to all of the pulse-alone trials, excluding the first and last blocks of five pulse-alone trials each. For brevity, the main effects of prepulse intensity (which were always significant) are not discussed here. Data from the no-stim trials are not included in the examples because the values obtained were negligible relative to values from trials containing startle stimuli.
***(ix) Induction of psychological stress.*** Mice in the experimental groups were exposed for 10 minutes to thoroughly soiled cat litter (used by a cat for 2 days and sifted for faeces). Control (WT) mice were exposed for the same time to unused litter.
   ***Behavioral testing:***
***(x) Elevated plus-maze (EPM).*** The maze we used was a black opaque perspex platform with four arms in the shape of a plus, elevated 78 cm above the ground, as described by File (Griebel et al., 1995). Each arm was 24 cm long and 7.5 cm wide. One pair of opposite arms was "closed", i.e., the arms were enclosed by 20.5-cm-high perspex walls on both sides and on the outer edges of the platform, and the other pair was "open", surrounded only by a 3-mm-high perspex lip, which served as a tactile guide for animals in the open areas. The apparatus was illuminated by dim red lighting that provided 40-60 lux in both the open and the closed arms. Mice were placed one at a time in the central platform, facing towards different arms on different days in randomized order. Between each test session the maze was cleaned with an aqueous solution of 5% ethanol and dried thoroughly.
   Behavior on the EPM was recorded using EthoVision programs (Noldus) that recorded the location of the mouse over the 5-minute test period. To ensure that the software provided accurate monitoring of the various parameters selected for analysis, videotaped replay of the behavior of randomly chosen mice was scrutinized by an experienced observer.
   Five behavioral parameters were assessed: (1) time spent in the open arms; (2) time spent in the closed arms; (3) number of entries into open arms; (4) number of entries into closed arms; (5) total number of entries into all arms. Mice were recorded as having entered an open or closed arm only when all four paws had passed over the dividing line between open and closed arms. The number of entries into any arm of the maze (total arm entries) was defined as 'exploration activity'.
***(xi) Acoustic startle response (ASR).*** Pairs of mice were tested in startle chambers. The ASR and pre-pulse inhibition were measured using two ventilated startle chambers (SR-LAB system, San Diego Instruments, San Diego, CA). Each chamber consists of a Plexiglas cylinder resting on a platform inside a ventilated sound-attenuated chamber. A high-frequency loudspeaker inside the chamber produces both a continuous broad-band background noise of 68 dB and different acoustic stimuli. Movement inside the tube is detected by a piezoelectric accelerometer located below the frame. The amplitude of the ASR of the whole body to an acoustic pulse was defined as the average of 100 accelerometer readings, 100 ms each, collected from pulse onset. These readings (signals) were digitized and stored in a computer. Sound levels within each test chamber are routinely measured using a sound-level meter (Radio Shack, San Diego Instruments) to ensure consistent presentation. An SR-LAB calibration unit was used routinely to ensure consistency of the stabilimeter sensitivity between test chambers and over time (Swerdlow and Geyer, 1998). The mice were placed inside the tube, and the startle session started with a 5-minute period of acclimatization to the background noise level of 68 dB, which was maintained throughout the session.
***(xii) Study design for determination of "cut-off behavioral criteria" (CBC).***
   The studies were designed in two steps:
   Step I - Prior to attempting to distinguish the differentially affected subgroups, we routinely perform a preliminary assessment of the overall response of the exposed population intended to ascertain the accuracy of our zero-hypothesis, i.e. to demonstrate that exposure to the stressor did in fact have significant overall behavioral effects on the exposed animals as a group compared to controls, in each of our studies. The data are also ascertained to demonstrate a range of varying degrees of behavioral changes.
   Behavioral changes, such as extremely compromised exploratory behavior on the plus maze and markedly increased startle reaction that does not undergo any adaptation reflect anxiety-like behaviors, i.e. fearfulness and hypervigilance. In keeping with the work of Blanchard and Blanchard (Blanchard et al., 1990; Blanchard et al., 1993; Blanchard et al., 1998), Adamec (Adamec et al., 1998; Adamec et al., 1999a) and Cohen (Cohen et al., 1996; Cohen et al., 2000; Cohen et al., 2003) the observed behaviors at this time-point are considered to reflect relatively long-term and persistent changes. Since it has as yet not been possible to design an animal model for the intrusive cluster of symptoms, changes such as these, which persist over the space of a week or more, are considered to represent a fair representation of PTSD-like symptoms in terms of animal models.
   Step II - The CBC applied to exposed animals:
   Having established that the stressor had an effect on the animals and that not all animals responded to it in the same manner, we focus only on animals that demonstrate extremes of behavioral change on the one hand or virtually no change on the other.
   In order to maximize the clarity of which animals to define as "affected", and so to minimize the chance of including "false positives", we define the behavioral cut-off criteria to represent the most extreme degree of behavioral disturbance in each of two consecutive behavioral paradigms. In order to be defined as "affected", the individual animal has to have conformed to both sets of criteria, consecutively. Conversely, in order to be considered to have responded hardly at all, animals must conform to equally extreme criteria for "near normal" behaviors. The validity of the criteria are re-affirmed in each study by ascertaining that the vast majority of unexposed control animals conform to the latter and none, or almost none, to the former. The CBCs determined as above, were as follows: **(a) Maladapted:** 1) Five minutes spent in closed arms and zero (0) entries into the open-arms; 2) Mean amplitude of the startle response (at 110 Db) > 800 units and nonhabituation of the acoustic startle response; **(b) Well-Adapted:** 1) 0-1 minutes spent in closed arms and ≥ 8 open-arm entries; 2) Mean amplitude of the startle response (at 110 Db) < 600 units and normal habituation of the acoustic startle response.
***(xiii) Supplemental Information.*** The behavioral outcome in mice exposed to the odor of a predator has been used as a model for PTSD (Adamec et al., 1999b; Cohen et al., 2003). Brief, escapable exposure of mice or rats to a cat or cat odor increases the defensive behaviors observed in a visible burrow system for many hours after removal of the threat (Rodgers et al., 1990). The long-lasting behavior abnormality is being viewed as maladaptation to the predator stress (i.e. PTSD). The stressor and the time scale used in the present study might justify view the results as relevance to PTSD according to the following criteria (Yehuda and Antelman, 1993): (a) The stressor is strong and transient and provides a more natural setting than that offered by other types of stressors, such as electrical shocks to the tail (Adamec et al., 1997). (b) The observed reduction in time spent by the stressed mice in the open arms of the EPM is reminiscent of the avoidance behavior seen in patients with PTSD. Stressed mice did not show fewer total entries in the EPM than unstressed mice. This finding is consistent with anxiety rather than with nonspecific impairment of locomotion. The DSM-IV defines this symptom as the persistent avoidance of reminders of the trauma and the numbing of responsiveness. Since the traumatic event in these mice took place in an open space, it is in line with this definition. (c) Seven days in the life of a mouse that normally lives for 3 years is roughly equivalent to 6 months in a human life span of 72 years (Adamec et al., 1997). It thus seems that our assessment of mice 7 days after the trauma indeed points to PTSD rather than to an acute stress reaction.
***(xiv) Antibodies and reagents.*** Mouse recombinant IL-2 (mrIL-2) and anti mouse ζ-CD3 (anti-CD3; clone 145-2C11) were purchased from R&D Systems (Minneapolis, MN). Rat anti-mouse phycoerythrin (PE)-conjugated CD25 antibody (PC61) was purchased from Pharmingen (Becton-Dickinson, Franklin Lakes, NJ).
***(xv) Histology and immunohistochemistry of paraffin-embedded brain sections.*** Paraffin-embedded brain tissues from maladapted nude mice replenished with a normal population of wild-type splenocytes, or from well-adapted nude mice replenished with wild type splenocytes depleted of Treg cells, were cut into 4-µm-thick coronal sections, deparaffinized with xylene, and dehydrated with a graded series of ethanol solutions. The sections were then stained with Luxol fast blue (Sigma-Aldrich, Israel) and counterstained with Fast Red (Sigma, Israel). For immunohistochemical analyses, deparaffinized and dehydrated sections were immersed (30 min) in methanol containing 3% H₂O₂ and 1% of concentrated HCl to block endogenous peroxidase activity, treated (1 h) with phosphate-buffered saline (PBS), pH 7.4, containing 20% normal rabbit serum and 0.3% Triton X-100, and incubated overnight at room temperature with anti-CD3 antibodies (Serotec, Oxford, UK; diluted 1:50 in PBS containing 2% normal rabbit serum). The sections were washed with PBS and incubated for 30 min, first with biotinylated anti-rabbit IgG and then with avidin-biotinylated peroxidase complex (Vector Laboratories, Burlingame, CA). Peroxidase activity in a solution of 3,3'-diaminobenzidine was visualized by light microscopy.
***(xvi) Preparation of splenocytes.*** Donor splenocytes from rats (aged up to 10 weeks) were obtained by rupturing the spleen and following conventional procedures. The splenocytes were washed with hypotonic buffer (ACK) to lyse red blood cells.
***(xvii) Preparation of lymphocytes.*** Donor mice lymph nodes (axillary, inguinal, superficial cervical, mandibular, and mesenteric) were ruptured through mesh. The lymphocytes were washed with hypotonic buffer (ACK) to lyse red blood cells.
***(xviii) Purification of CD4⁺CD25⁺ and CD4⁺CD25⁻ T cells.*** Lymph nodes were harvested and mashed. T cells were enriched by negative selection and purified on CD3-cell columns (MTCC-25; R&D Systems). The enriched T cells were incubated with anti-CD8 microbeads (Miltenyi Biotec, Bergisch Gladbach, Germany), and negatively selected CD4⁺ T cells were incubated with PE-conjugated anti-CD25 (30 pg/10^{g} cells) in PBS/2% fetal calf serum. They were then washed and incubated with anti-PE microbeads (Miltenyi Biotec) and subjected to magnetic separation with AutoMACS (Miltenyi Biotec). The retained cells were eluted from the column as purified CD4⁺CD25⁺ cells. The negative fraction consisted of CD4⁺CD25⁻ T cells. Cell purity was checked by FACSort (Becton-Dickinson) and typically ranged from 88% to 95%. Purified cells were cultured in 24-well plates (1 ml).
***(xix) Activation of CD4*⁺*CD25*⁺ *regulatory T cells.*** Purified regulatory T cells (Treg; 0.5x10⁶/ml) were activated in RPMI medium supplemented with L-glutamine (2 mM), 2-mercaptoethanol (5×10⁻⁵ M), sodium pyruvate (1 mM), penicillin (100 IU/ml), streptomycin (100 µg/ml), non-essential amino acids (1 ml/100 ml), and autologous serum 2% (vol/vol) in the presence of mrIL-2 (5 ng/ml) and soluble anti-CD3 antibodies (1 ng/ml). Irradiated (2500 rad) splenocytes (1.5x10⁶/ml) were added to the culture. Cells were activated for 24 or 96 hr.
***(xx) Inhibition assay (co-culturing of Teff with Treg).*** Naïve effector T cells (Teff; 50x10³ /well) were co-cultured with decreasing numbers of activated Treg for 72 h in 96-well flat-bottomed plates in the presence of irradiated splenocytes (10⁶/ml) supplemented with anti-CD3 antibodies. [³H]-thymidine (1 µCi) was added for the last 16 hr of culture. After the cells were harvested, their analyzed [³H]-thymidine content was analyzed by the use of a gamma counter.

### Example 1. Vaccination with Cop-1 has an anti-psychotic effect and protects against sensorimotor dysfunction induced by psychotomimetic agents

Dizocilpine maleate ((+)MK-801, an antagonist of the N-methyl-D-aspartate (NMDA) receptor channel) and AMPH act as psychotomimetic agents, inducing - via neurotransmitter imbalance - psychotic symptoms in healthy individuals and exacerbating psychotic symptoms in schizophrenic patients (Lahti et al., 2001). We therefore used these two compounds in an animal model to induce psychotic behavior that simulates behavioral and cellular abnormalities associated with schizophrenia (Tenn et al., 2003).

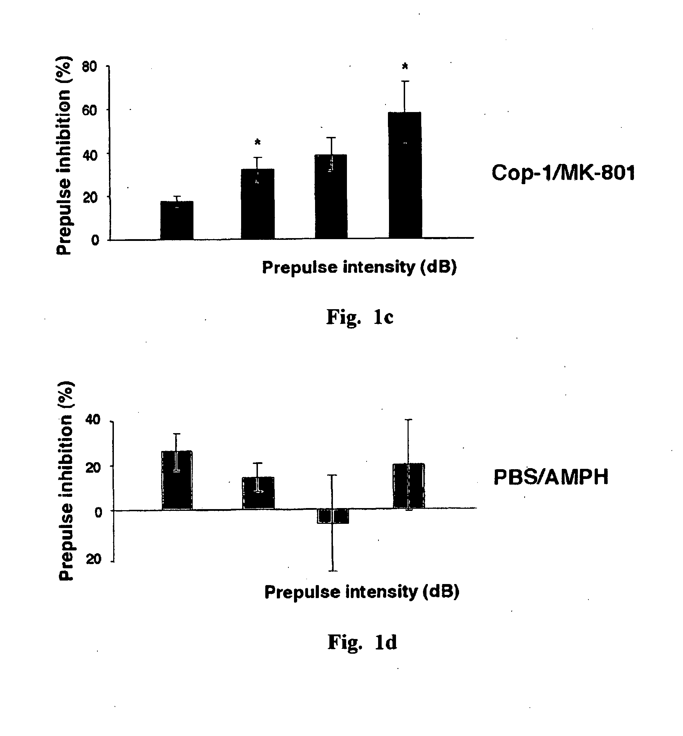

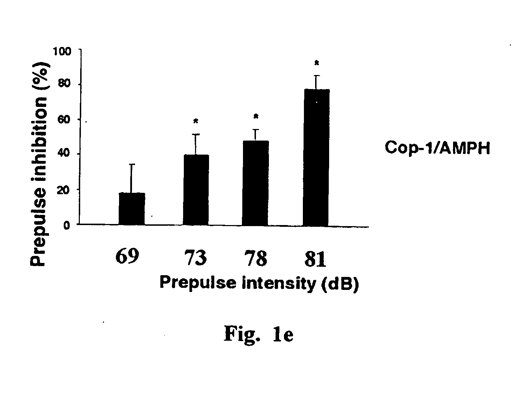

The neurotransmitter imbalance induced by MK-801 or AMPH also causes sensorimotor dysfunction, another characteristic feature of patients with schizophrenia. Because (as shown below) T cell-based therapy counteracted the effect of these drugs on cognition, we assumed that other functions impaired by these drugs would be similarly affected. Sensorimotor gating can be assessed experimentally by drug-induced PPI of the acoustic startle response. One week before administration of MK-801 or AMPH, C57BL/6J mice were either inoculated with Cop-1/CFA or with PBS/CFA. The psychotomimetic drugs were injected 15 min before measurement of the PPI (Van den Buuse et al., 2003). As expected, PPI in untreated normal mice increased with increasing prepulse intensity (Fig. 1a) whereas, in mice injected with MK-801 (C57BL/6J) or AMPH (Fig. 1d) the PPI response was abnormal. Vaccination with Cop-1/CFA, but not with PBS/CFA, prevented the abnormal behavior induced by MK-801 (Figs. 1b, 1c). Similar results were obtained with AMPH with (Fig. 1d) and without Cop-1 immunization (Fig. 1e). Thus, sensorimotor dysfunction induced by administration of each of the psychotomimetic agents was prevented or partially restored by vaccination with Cop-1/CFA.

Due to the rapid onset of symptoms that occurs in the experimental paradigm used here, the mice had to be vaccinated before psychosis was induced. Further experiments with a wider window for therapeutic intervention have indeed shown that vaccination with Cop-1 after exposure of rats to psychotomimetic drugs was beneficial (not shown).

### Example 2. Cognitive functions are impaired in the absence of T cells

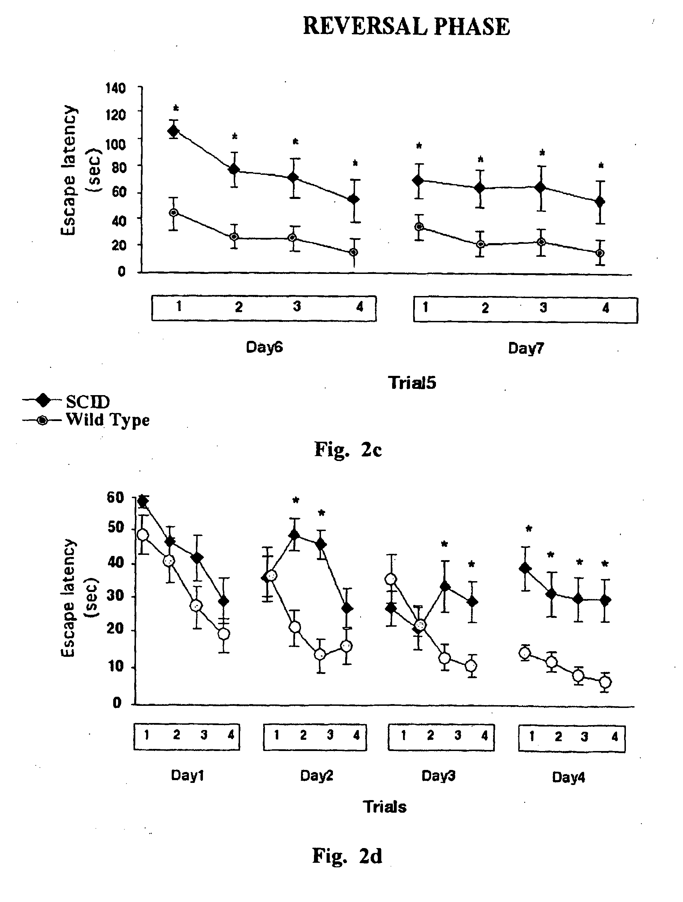

To establish whether learning and memory processes are dependent on the integrity of the immune system, we compared the spatial learning/memory of wild-type and SCID BALB/c/OLA mice, by using the MWM, a hippocampal-dependent visuo-spatial learning/memory task: The SCID mice manifested significant impairment of spatial memory compared with their wild-type counterparts (Figs. 2a-2c). During the acquisition (Fig. 2a), extinction (Fig. 2b), and reversal (Fig. 2c) phases of the MWM task, mice devoid of adaptive immunity showed significantly increased latency in finding the hidden platform compared with wild-type mice (Figs. 2a-2c). Unlike the wild-type, the immune-deficient (SCID) mice failed to recall data from the previous day's training trial (Figs. 2a, 2c). Moreover, the SCID mice started out at a lower level of performance than the wild-type, indicating that their general skills in carrying out the task were impaired, at least to some extent (Fig. 2a). The two groups did not differ in their performance of the visual platform task, or in a test in which distal cues were removed and the mice were tested once a day for 4 days, or in their swimming strategies, distance, or speed (data not shown).

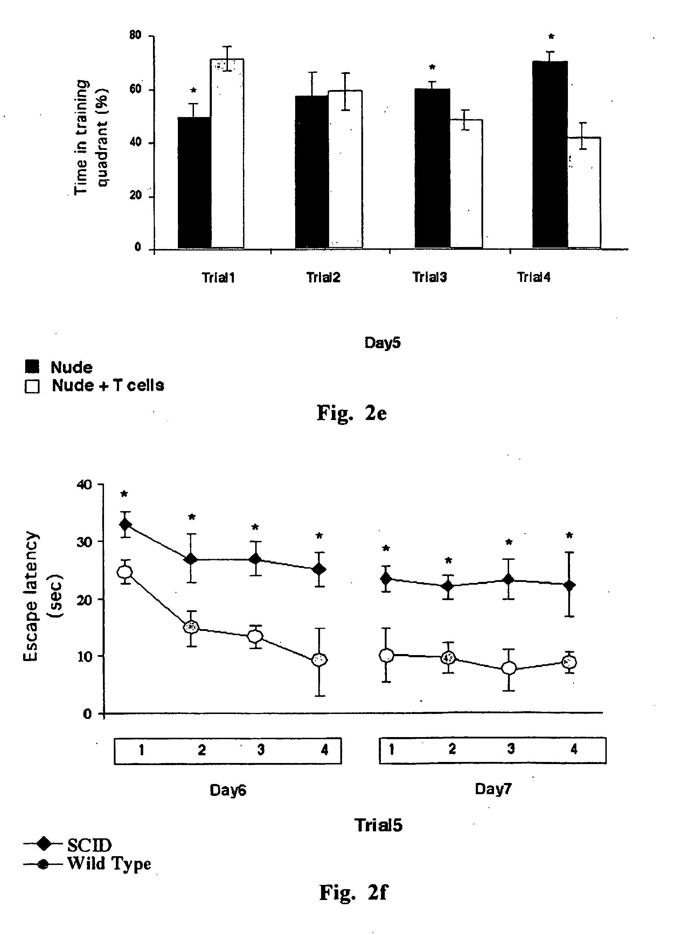

It should be emphasized that, in the above set of experiments, we used SCID mice (deficient in both T cell and B cell responses) rather than nude mice (deficient only in mature T cells) to exclude differences that might be attributable to absence of fur (in nude mice) rather than to the mere differences in immune system activity. The immune deficiency of SCID mice, however, is a result of knockout of RAG 1/2 genes. Because RAG1 is expressed in brains of normal mice (Kim et al., 2003) (although their functions there are still unknown), it was necessary to further substantiate our conclusion that the observed difference between the SCID and the wild-type mice was attributable to T cell immunity. We therefore compared nude mice replenished with a normal T cell population with nonreplenished nude mice (Fig. 2). We replenished nude mice with T cells from matched wild-type mice and tested them 3 weeks later on the MWM task (Figs. 2d-2f). During the acquisition and the reversal phases, nude mice replenished with T cells showed significantly shorter latency in finding the hidden platform than did nonreplenished nude mice. In the extinction phase, the replenished mice needed to spend significantly less time than the nonreplenished mice in the training quadrant for the trials. Moreover, the nonreplenished mice were significantly less able to recall data from the previous day's training trial and showed significantly slower rates of learning than those of their replenished counterparts (Figs. 2d-2f).

### Example 3. Cop-1 vaccination is protective against cognitive impairment induced by psychotomimetic agents

The above results encouraged us to examine the possibility that, in mice with impaired cognitive functions caused for example by neurotransmitter imbalance, boosting of the relevant T cells (e.g., by T cell-based vaccination) might have a therapeutic effect.

As mentioned above, MK-801 and AMPH induce in humans psychotic symptoms. In mice, such symptoms evidently simulate the cognitive impairment and behavioral abnormalities associated with schizophrenia (Tenn et al., 2003). A number of authors have reported an MK-801-induced deficit in acquisition of spatial memory (Whishaw et al., 1989; Ahlander et al., 1999) and nonspatial memory tasks (Griesbach et al., 1998).

To examine whether T cell-based vaccination can overcome behavioral abnormalities and cognitive impairment resulting from neurotransmitter imbalance, we immunized mice with Cop-1. This synthetic antigen apparently acts as a weak agonist of a wide range of self-reactive T cells, thereby stimulating a response that is mediated by T cells that crossreact with CNS antigens and is needed to fight off neurodegenerative conditions (Kipnis et al., 2000). It should be emphasized that, unlike myelin-associated and other CNS-associated antigens, antigens (such as ovalbumin) that do not crossreact with CNS-specific proteins fail to accumulate in the healthy brain and have no protective effect (Kipnis et al., 2000; Moalem et al., 2000).

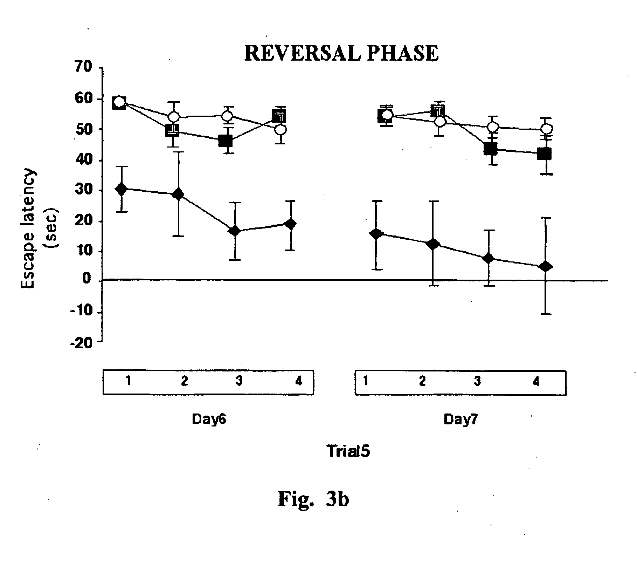

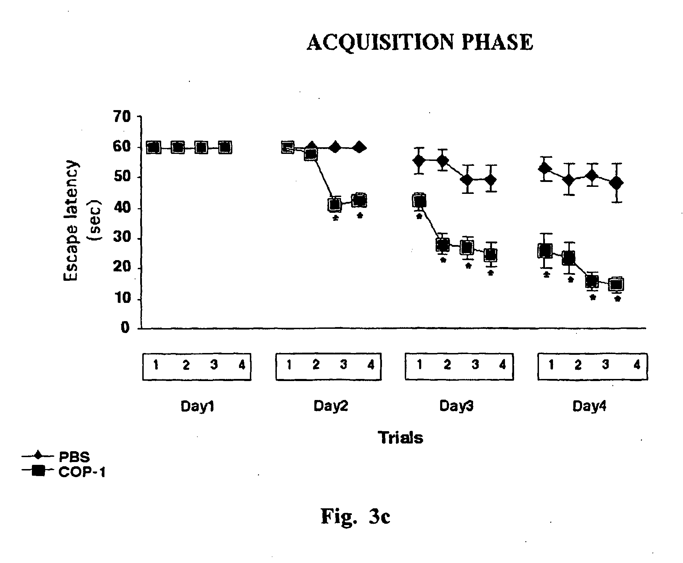

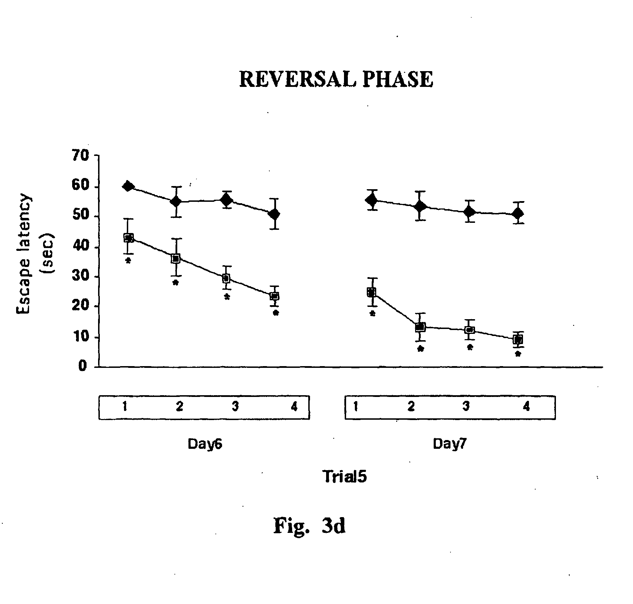

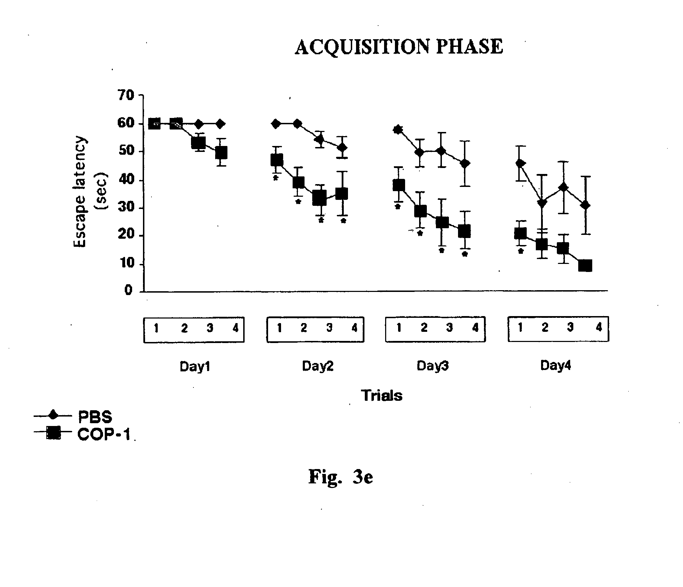

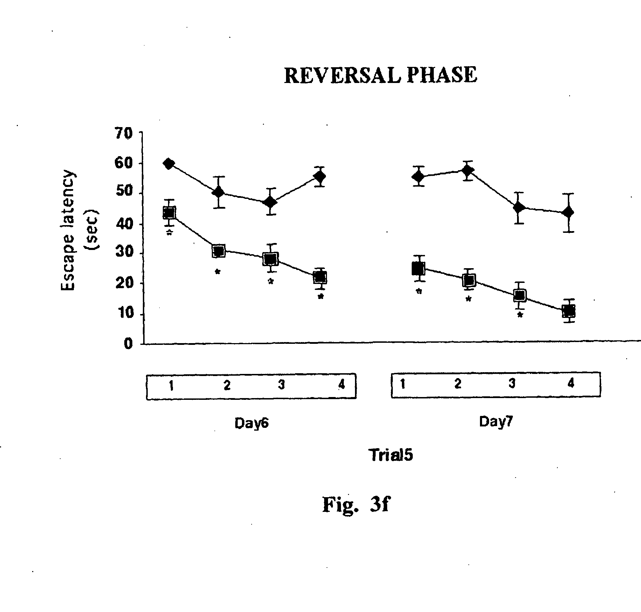

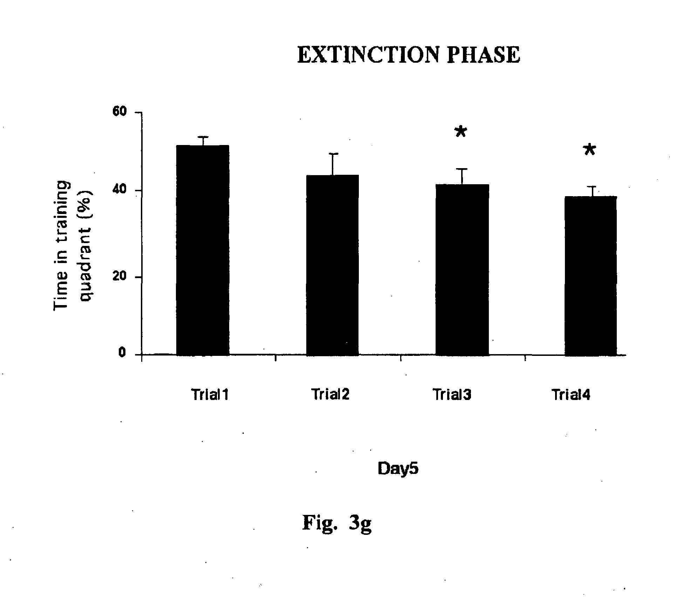

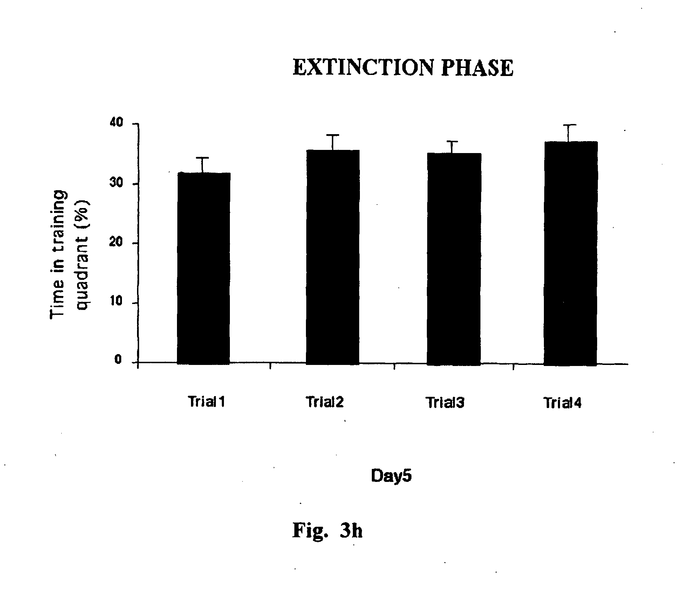

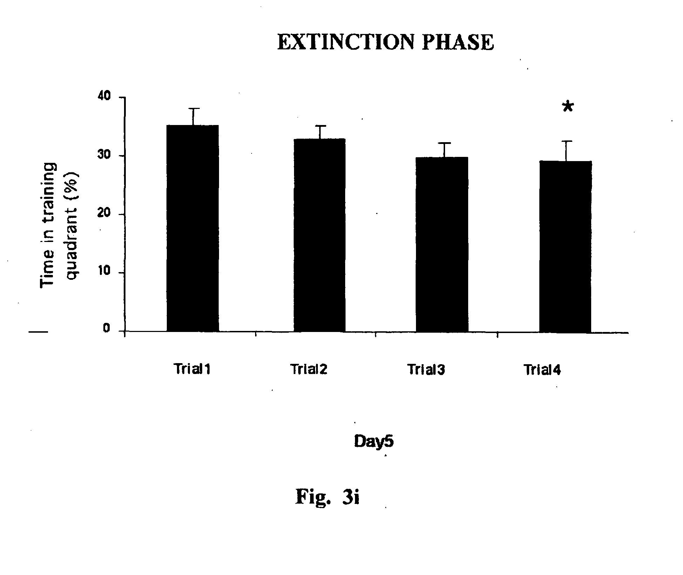

One week before receiving the psychotomimetic drug, C57BL/6J mice were immunized with Cop-I emulsified in CFA or with PBS emulsified in CFA. Relative to behavior in naive normal mice, performance of a task requiring spatial learning and memory in the MWM was significantly impaired in mice injected with MK-801 or AMPH (Figs. 3a, 3b), as indicated by their significantly higher escape latencies. During the acquisition (Figs. 3c, 3e) and the reversal (Figs. 3d, 3f) phases of the MWM task, PBS/CFA injected mice that had received either of the psychotomimetic drugs took significantly longer than the corresponding Cop-1/CFA vaccinated mice to acquire the spatial navigation task, if they were able to acquire it at all. In the extinction phase, naive normal mice showed a decrease over successive trials in the time spent in the training quadrant (Fig. 3g). Injection of MK-801 weakened this characteristic feature in mice immunized with PBS/CFA (Fig. 3h), but not in mice immunized with Cop-1/CFA (Fig. 3i). Similar results were obtained in amphetamine-injected mice (data not shown).

The Cop-1/CFA-vaccinated mice injected with each of the psychotomimetic drugs learned to swim to the hidden platform and make use of it as a refuge by climbing onto it and remaining there, as indicated by decreasing latencies in successive trials. In contrast, when the corresponding PBS/CFA-injected mice encountered the hidden platform, they behaved in an abnormal and maladaptive way. Even when placed directly on the hidden platform after a trial in which they had failed to locate it, these mice quickly walked or jumped off and continued swimming in a haphazard and disorganized manner. In all of these tasks, the behavior of normal naïve mice and of normal CFA/PBS-injected mice was identical (data not shown).

Fig. 4 depicts the behavior of naive normal mice and of MK-801-treated mice immunized with Cop-1/CFA or PBS/CFA, and shows that Cop-1/CFA immunized mice, unlike the PBS/CFA immunized mice, adopted methodical swimming strategies similar to those seen in normal mice. Thus, Cop-1/CFA-vaccinated mice injected with a psychotomimetic drug learned to swim away from the wall to search for the platform in the inner part of the pool and to use the platform as a refuge when they found it. In contrast, the behavior of the PBS/CFA-treated mice injected with a psychotomimetic drug showed severe disturbances, including hyperactivity, swimming over the platform, and aimless swimming in circles. Their subsequent performance in the elevated-plus-maze task indicated, however, that the observed differences in spatial learning ability in the MWM between these two groups of mice was not caused by a difference in anxiety. Furthermore, in the social behavior test, the mice that were vaccinated with Cop-1 also showed better communicative behavior than the controls (data not shown).

### Example 4. Strain dependence and T-cell dependence on the ability to withstand psychological stress

Protection against neurodegenerative conditions in the CNS is T-cell dependent. Psychological trauma, like physical CNS insult, can cause widespread long-term changes in neurological and neurohormonal functioning, and appears to be related to structural changes (Markowitsch et al., 1998; Myhrer, 1998). There is evidence that mental/emotional state directly affects the immune system status (de Groot et al., 2002; McEwen, 2002; Dhabhar and McEwen, 1999). It was therefore of interest to examine the effect of CD4+ (adaptive immunity) T cells on the ability to withstand psychological trauma.

Here we examined whether T cells also play a role in the ability of mice to withstand psychological stress (caused, for example, by predator odor) associated with behavioral changes reminiscent of post-traumatic stress disorder (PTSD). Previous studies have shown that exposure of rats or mice to a predator (cat) or odor of a predator (thoroughly soiled cat litter) for 10 minutes causes major stress in these animals (Adamec et al., 1997, 1999a, 1999b; Cohen et al., 1996, 2000, 2003). This stress model was used herein.

Measurement of behavioral adaptation (acoustic startle response and avoidance behavior) in mice after their exposure to predator odor revealed that maladaptation was significantly more prevalent (χ²=10.6, *P*<0.001) in immune-deficient mice (62%) than in their wild-type counterparts (17%). The prevalence of maladaptation in the normal mice was reduced upon removal of naturally occurring Treg cells, which normally suppress autoimmunity. The ability to cope with stress was correlated with recruitment of T cells in the brain. These findings suggest that a well-controlled T cell-dependent dialog between the brain and the immune system is needed for *mens sana in corpore sano.*

We first exposed naive adult mice of two strains (C57B1/6J and BALB/c) to the odor of a cat, as previously described (Cohen et al., 2003). Seven days later we assessed their behavioral responses to two sequentially administered behavioral challenges, the elevated plus-maze (EPM) and the acoustic startle response (ASR), which together provide a framework for selected cutoff behavioral criteria (CBC). By classifying the tested mice as either "maladapted" or "well adapted", we could determine the prevalence of the more severely affected animals.

The two strains (C57B1/6J and BALB/c) differed in their overall adaptation to the imposed psychological trauma (Figs. 5a-5c). The incidence of maladaptation in C57B1/6J mice was 36.8%, whereas in BALB/c mice it was only 10.5% (χ²=3.7, *P* < 0.05; Fig. 5a). The differences were significantly manifested in the ASR (Fig. 5b), but not in the time spent in the closed arms of the EPM (Fig. 5c). This finding is in line with previous observations in connection with the strain-related ability of mice to withstand glutamate toxicity and optic nerve injury (Kipnis et al., 2001).

The results prompted us to investigate whether the adaptive immune system (represented by CD4+ T cells) affects the behavioral consequences of traumatic mental stress and the adaptation to such stress. We therefore examined the ability to adapt to the psychological stress in the absence of well-functioning immune system. Because of the relatively low incidence of maladaptation in the BALB/c mice (Fig. 5a) we used this strain to compare the response to stress in the wild-type (WT) to that in mice with severe combined immune deficiency (SCID) with the same genetic background. Significantly more mice showed symptoms of maladaptation in the SCID mice than in the WT (61.9% compared to 17.2%; χ² =10.6, *P* < 0.001; Fig. 5d. The same comparison between nude mice (devoid of mature T cells only) and the WT data yielded similar results (70% compared to 17.2%; χ² =13.9, *P*<0.0002; Fig. 5d, verifying that the observed differences were due to the absence of mature T cells. Differences between the WT mice and both the SCID and the nude mice were significantly manifested in the ASR (Fig. 5e) as well as in the time spent in the closed arms of the EPM (Fig. 5f).

### Example 5. Behavioral adaptation to psychological stress.

Fig. 6 shows the ability of mice immunized with Cop-1 to withstand psychological stress in comparison with PBS-treated mice (control), after a single single 10-min exposure to the odor of a predator. It can be seen that this single exposure caused behavioral changes in 40.8% of male C57B1/6J control mice (immunized with PBS emulsified in CFA) and in only 14.8% of Cop-1 immunized mice. Maladaptation, resembling PTSD symptoms. was more prevalent in control mice than in Cop-1 immunized mice (P<0.05), verifying that the observed differences were attributable to the presence of protective Cop-1 reactive T cells.

### Example 6. Production of BDNF by Cop-1-reactive T cells upon encountering brain proteins.

Several research groups have reported that T cells reactive to Cop-1 home to sites of pathology in the CNS (Kipnis et al., 2000; Aharoni et al., 2002), and that activated Cop-1-reactive T cells, being able to cross-react with various CNS-related self-antigens, can produce neurotrophic factors such as BDNF (Kipnis et al., 2000; Aharoni et al., 2002; Kerschensteiner et al., 2003), which is known for its ability to confer neuroprotection to injured CNS tissue. BDNF deficiency has been reported in patients with schizophrenia (Weickert et al., 2003, Egan et al., 2003); it is unclear, however, whether the deficiency is a cause or an effect, and whether BDNF-based treatment will be beneficial.

Production of neurotrophic factors by T cells depends on the state of activation of the T cells (Moalem et al., 2000). Production of neurotrophic factors by Cop-1-reactive T cells therefore evidently requires a local signal from resident antigen-presenting cells that these T cells can recognize. We therefore carried out an experiment *in vitro* to determine whether Cop-1-reactive T cells, on encountering CNS myelin, can produce BDNF.

Cop-1-reactive T cells were cultured for 48 h with Cop-1, MBP, ovalbumin, or concanavalin A in stimulation medium. T cell supernatants were collected and subjected to sandwich ELISA. Table 2 shows that the production of BDNF by Cop-1-reactive T cells was increased when these T cells encountered not only their specific antigen (Cop-1) but also the CNS-related self-antigen MBP. Compared with unstimulated T cells, secretion of BDNF by Cop-1-reactive T cells was significantly increased after stimulation of the T cells with a specific antigen (Cop-1) or with a self-antigen (MBP) that crossreacts with Cop-1. Values are mean values (pg/ml) +/- SE (from three independent experiments) of the amounts of BDNF secreted by Cop-1-specific T cells in response to stimulation by various antigens.

**Table 2. ELISA of BDNF secreted by Cop-1-reactive T cells**

| Antigen | - | Cop-1 | MBP | OVA | Con A |
|---|---|---|---|---|---|
| pg/ml | 400 +/- 45 | 1220 +/- 100 | 1500 +/- 150 | 440 +/-50 | 1100 +/- 120 |

### Example 7. Naturally occurring CD4⁺CD25⁺ regulatory T cells (Treg) suppress the ability to withstand psychological stress

The spontaneous ability to fight off the sequelae of a mechanical (e.g. crush) injury or a biochemical insult (e.g. from glutamate toxicity) to the CNS (Kipnis et al., 2002) is suppressed by naturally occurring Treg, which comprise approximately 10% of the CD4⁺ T-cell population (Shevach, 2000). These cells were shown to suppress ability to fight off degenerative conditions in the CNS imposed for example by axonal injury (Kipnis et al., 2002).

To address the possibility that these cells control the spontaneous ability to fight off mental stress, we compared nude BALB/c mice with splenocytes obtained from WT mice depleted of Treg (devoid of Treg) to nude mice replenished with a normal splenocyte population (i.e., including Treg). In nude mice replenished with splenocytes free of Treg the prevalence of maladaptation was significantly lower (20%) than in the mice replenished with a normal T-cell population containing both Treg and effector T cells (50%) (χ²=4.0, P<0.046 (Fig. 7a). Significant differences between the two groups were observed both in the ASR (Fig. 7b) and in the time spent in the closed arms of the EPM (Fig. 7c).

### Example 8. T-cell accumulation in the brains of stressed mice correlates with behavioral adaptation

In mice and rats suffering from neurodegenerative conditions, the beneficial effect of T cells is correlated with accumulation of T cells at the site of the lesion (Butovsky et al., 2001; Hauben et al., 2000). To determine the relationship between the observed beneficial effect of the T-cell response and the consequences of exposure to stressful psychological conditions we examined whether it involved homing of T cells to the CNS. This was done by comparing the immuno-cytochemical staining for T cells in brain slices obtained from mice replenished with splenocytes depleted of Treg with those from mice replenished with a whole splenocyte population. Staining of brain slices with hematoxylin and eosin revealed no structural alterations in the hippocampus or amygdala (data not shown) between these two groups and the WT mice. Luxol fast blue staining for myelin reactivity also showed no differences between maladapted (Figs. 8ai, 8aiii) and well-adapted mice (Figs. 8bi, 8biii) compared to the WT (Fig. 8ci). Staining with anti-CD3 antibodies, however, revealed large numbers of T cells in these brain regions of well-adapted mice (Figs. 8aii, 8aiv) and hardly any in maladapted (Figs. 8bii, 8biv) or normal WT mice (Fig. 8cii), suggesting that the recruitment of T cells to the brain is correlated with the resistance to mental stress. We wish, however, to emphasize that accumulation of T cells altogether is modest and by no means imply that PTSD can benefit from inflammation, rather that a well-controlled adaptive immunity assists in resisting consequences of mental stress, similarly to physical stress, and through the same mechanism.

### Example 9. Cop-1 alleviates the suppressive activity mediated by Treg

Naïve Teff cells (50x10³ cells/well) were co-cultured with decreasing numbers (50, 25, 12.5 and 6.5x10³ cells/well) of Treg cells that have been activated for 24 h with anti-CD3 and mrIL-2. The activation of the Treg cells was carried out in the absence of Cop-1 (control) or, after 24 h, activated Treg cells were incubated for 2 h with Cop-1 (20 µg/ml in PBS) before co-culturing them with Teff, and then Cop-1 (20 µg/ml) was added to the co-cultures of Teff and Treg (Tregcop+cop) and the co-cultures were further incubated. Fig. 9 shows that incubation of the activated Treg (reg) for 2 h with Cop-1 prior to their co-culturing with Teff (eff) and further incubation of the co-culture with Cop-1 alleviated the suppression of Teff compared to the control. The proliferation of Teff also increased with decreasing concentrations of activated Treg. T cell proliferation was assayed by incorporation of [³H]-thymidine into effector T cells co-cultured with Treg. Recorded values are from one representative experiment out of three and are expressed as means ± SD of 4 replicates.

### REFERENCES

Adarriec RE, Shallow T, Budgell J. Blockade of CCK(B) but not CCK(A) receptors before and after the stress of predator exposure prevents lasting increases in anxiety-like behavior: implications for anxiety associated with posttraumatic stress disorder. Behav Neurosci 111:435-49 (1997)
Adamec R, Kent P, Anisman H, Shallow T, Merali Z. Neural plasticity, neuropeptides and anxiety in animals--implications for understanding and treating affective disorder following traumatic stress in humans. Neurosci Biobehav Rev 23:301-18 (1998)
Adamec RE, Burton P, Shallow T, Budgell J. NMDA receptors mediate lasting increases in anxiety-like behavior produced by the stress of predator exposure-implications for anxiety associated with posttraumatic stress disorder. Physiol Behav 65: 723-37 (1999a)
Adamec RE, Burton P, Shallow T, Budgell J. Unilateral block of NMDA receptors in the amygdala prevents predator stress-induced lasting increases in anxiety-like behavior and unconditioned startle-effective hemisphere depends on the behavior. Physiol. Behav. 65: 739-51 (1999b)
Aharoni, R., Meshorer, A., Sela, M. & Arnon, R. Oral treatment of mice with copolymer 1 (glatiramer acetate) results in the accumulation of specific Th2 cells in the central nervous system. J Neuroimmunol 126, 58-68 (2002)
Ahlander, M., Misane, I., Schott, P.A. & Ogren, S.O. A behavioral analysis of the spatial learning deficit induced by the NMDA receptor antagonist MK-801 (dizocilpine) in the rat. Neuropsychopharmacology 21, 414-26 (1999)
Amital, H. & Shoenfeld, Y. Autoimmunity and schizophrenia: an epiphenomenon or an etiology? Isr J Med Sci 29, 593-7 (1993)
Angelov, D.N. et al. Therapeutic vaccine for acute and chronic motor neuron diseases: implications for amyotrophic lateral sclerosis. Proc Natl Acad Sci U S A 100, 4790-5 (2003)
Blanchard RJ, Blanchard DC, Rodgers J, Weiss SM. The characterization and modeling of antipredator defensive behavior. Neurosci. Biobehav. Rev. 14: 463-72 (1990)
Blanchard RJ, Shepherd JK, Rodgers RJ, Magee L, Blanchard DC. Attenuation of antipredator defensive behavior in rats following chronic treatment with imipramine. Psychopharmacology (Berl) 110: 245-53 (1993)
Blanchard RJ, Nikulina JN, Sakai RR, McKittrick C, McEwen B, Blanchard DC. Behavioral and endocrine change following chronic predatory stress. Physiol. Behav. 63: 561-9 (1998)
Brzustowicz, L.M., Hayter, J.E., Hodgkinson, K.A., Chow, E.W. & Bassett, A.S. Fine mapping of the schizophrenia susceptibility locus on chromosome 1q22. Hum Hered 54, 199-209. (2002)
Butovsky, O., Hauben, E. & Schwartz, M. Morphological aspects of spinal cord autoimmune neuroprotection: colocalization of T cells with B7--2 (CD86) and prevention of cyst formation. Faseb J 15, 1065-7 (2001)
Cohen H, Friedberg S, Michael M, Kotler M, Zeev K. Interaction of CCK-4 induced anxiety and post-cat exposure anxiety in rats. Depress Anxiety 4: 144-5 (1996)
Cohen H, Kotler M, Matar M, Kaplan Z. Normalization of heart rate variability in posttraumatic stress disorder patients following fluoxetine treatment: preliminary results. Isr. Med. Assoc. J. 2: 296-301 (2000)
Cohen H, Zohar J, Matar M. The relevance of differential response to trauma in an animal model of posttraumatic stress disorder. Biol. Psychiatry 53: 463-73 (2003)
de Groot, J., Boersma, W.J., Scholten, J.W. & Koolhaas, J.M. Social stress in male mice impairs long-term antiviral immunity selectively in wounded subjects. Physiol. Behav. 75, 277-85 (2002)
de Kloet, R.E. Hormones, brain and stress. Endocr Regul 37, 51-68 (2003)
Deutsch, S.I., Rosse, R.B., Schwartz, B.L. & Mastropaolo, J. A revised excitotoxic hypothesis of schizophrenia: therapeutic implications. Clin Neuropharmacol 24, 43-9 (2001)
Dhabhar, F.S. & McEwen, B.S. Enhancing versus suppressive effects of stress hormones on skin immune function. Proc. Natl. Acad. Sci. U S A 96, 1059-64 (1999)
Egan, M.F., Weinberger, D.R. & Lu, B. Schizophrenia, III: brain-derived neurotropic factor and genetic risk. Am J Psychiatry 160, 1242 (2003)
Falkai, P. et al. Influence of genetic loading, obstetric complications and premorbid adjustment on brain morphology in schizophrenia: a MRI study. Eur Arch Psychiatry Clin Neurosci 253, 92-9 (2003)
Fridkis-Hareli M, Aharoni R, Teitelbaum D, Amon R, Sela M, Strominger JL. Binding motifs of copolymer 1 to multiple sclerosis- and rheumatoid arthritis-associated HLA-DR molecules. J Immunol. 162(8):4697-4704 (1999).
Fridkis-Hareli M, Santambrogio L, Stem JN, Fugger L, Brosnan C, Strominger JL. Novel synthetic amino acid copolymers that inhibit autoantigen-specific T cell responses and suppress experimental autoimmune encephalomyelitis. J Clin Invest. 109(12):1635-43 (2002).
Griebel G, Blanchard DC, Jung A, Blanchard RJ. A model of 'antipredator' defense in Swiss-Webster mice: effects of benzodiazepine receptor ligands with different intrinsic activities. Behav. Pharmacol. 6: 732-745 (1995)
Griesbach, G.S., Hu, D. & Amsel, A. Effects of MK-801 on vicarious trial-and-error and reversal of olfactory discrimination learning in weanling rats. Behav Brain Res 97, 29-38 (1998)
Hauben E, Butovsky O, Nevo U, Yoles E, Moalem G, Agranov G, et al. Passive or active immunization with myelin basic protein promotes recovery from spinal cord contusion. J. Neurosci. 20: 6421-6430 (2000)
Javitt, D.C. Treatment of negative and cognitive symptoms. Curr Psychiatry Rep 1, 25-30 (1999)
Kerschensteiner M, Stadelmann C, Dechant G, Wekerle H, Hohlfeld R. Neurotrophic cross-talk between the nervous and immune systems: implications for neurological diseases. Ann. Neurol. 53: 292-304 (2003)
Kim S. K, Wang K. C, Hong S. J, Chung C. K, Lim S. Y, Kim Y. Y, Chi G, Kim C. J, Chung Y. N, Kim H. J. & Cho B. K. Epilepsy Res. 56, 175-183 (2003)
Kipnis J, Yoles E, Porat Z, Cohen A, Mor F, Sela M, Cohen IR, Schwartz M. T cell immunity to copolymer 1 confers neuroprotection on the damaged optic nerve: possible therapy for optic neuropathies. Proc. Natl. Acad. Sci. U S A 97: 7446-51 (2000)
Kipnis J, Yoles E, Schori H, Hauben E, Shaked I, Schwartz M. Neuronal survival after CNS insult is determined by a genetically encoded autoimmune response. J. Neurosci. 21, 4564-71 (2001)
Kipnis, J. & Schwartz, M. Dual action of glatiramer acetate (Cop-1) in the treatment of CNS autoimmune and neurodegenerative disorders. Trends Mol. Med. 8, 319-23 (2002)
Kipnis J, Mizrahi T, Hauben E, Shaked I, Shevach E, Schwartz M. Neuroprotective autoimmunity: naturally occurring CD4+CD25+ regulatory T cells suppress the ability to withstand injury to the central nervous system. Proc. Natl. Acad. Sci. U S A; 99: 15620-5. (2002)
Kipnis J, Nevo U, Panikashvili D, Alexandrovich A, Yoles E, Akselrod S, Shohami E, Schwartz M. Therapeutic vaccination for closed head injury. J. Neurotrauma 20(6):559-69, (2003)
Lahti, A.C., Weiler, M.A., Tamara Michaelidis, B.A., Parwani, A. & Tamminga, C.A. Effects of ketamine in normal and schizophrenic volunteers. Neuropsychopharmacology 25, 455-67 (2001).
Lewis, D.A., Pierri, J.N., Volk, D.W., Melchitzky, D.S. & Woo, T.U. Altered GABA neurotransmission and prefrontal cortical dysfunction in schizophrenia. Biol Psychiatry 46, 616-26 (1999)
Lieberman, J. et al. Longitudinal study of brain morphology in first episode schizophrenia. Biol Psychiatry 49, 487-99 (2001).
Markowitsch, H.J. et al. Psychic trauma causing grossly reduced brain metabolism and cognitive deterioration. Neuropsychologia 36, 77-82 (1998)
McCullumsmith, R.E. & Meador-Woodruff, J.H. Striatal excitatory amino acid transporter transcript expression in schizophrenia, bipolar disorder, and major depressive disorder. Neuropsychopharmacology 26, 368-75 (2002)
McEwen, B.S. Protective and damaging effects of stress mediators: the good and bad sides of the response to stress. Metabolism 51, 2-4 (2002)
Mizrahi T, Hauben E, Schwartz M. The tissue-specific self-pathogen is the protective self-antigen: the case of uveitis. J. Immunol. 169: 5971-7 (2002)
Moalem G, Leibowitz-Amit R, Yoles E, Mor F, Cohen IR, Schwartz M. Autoimmune T cells protect neurons from secondary degeneration after central nervous system axotomy. Nat. Med. 5: 49-55 (1999)
Moalem G, Gdalyahu A, Shani Y, Otten U, Lazarovici P, Cohen IR, et al. Production of neurotrophins by activated T cells: Implications for neuroprotective autoimmunity. J. Autoimmun 20: 6421-6430 (2000)
Myhrer, T. Adverse psychological impact, glutamatergic dysfunction, and risk factors for Alzheimer's disease. Neurosci. Biobehav. Rev. 23, 131-9 (1998)
Raison, C.L. & Miller, A.H. The neuroimmunology of stress and depression. Semin Clin Neuropsychiatry 6, 277-94 (2001)
Rodgers RJ, Blanchard DC, Wong LK, Blanchard RJ. Effects of scopolamine on antipredator defense reactions in wild and laboratory rats. Pharmacol Biochem Behav 36: 575-83 (1900)
Rothermundt M, Arolt V, Bayer TA. Review of immunological and immunopathological findings in schizophrenia. Brain Behav. Immun. 15: 319-339 (2001)
Rummel, C., Hamann, J., Kissling, W. & Leucht, S. New generation antipsychotics for first episode schizophrenia. Cochrane Database Syst Rev, CD004410 (2003)
Schori, H. et al. Vaccination for protection of retinal ganglion cells against death from glutamate cytotoxicity and ocular hypertension: Implications for glaucoma. Proc. Natl. Acad. Sci. U S A 98, 3398-403 (2001)
Schwartz, M., Cohen, 1., Lazarov-Spiegler, O., Moalem, G. & Yoles, E. The remedy may lie in ourselves: prospects for immune cell therapy in central nervous system protection and repair. J Mol Med 77, 713-7 (1999a)
Schwartz, M., Moalem, G., Leibowitz-Amit, R. & Cohen, I.R. Innate and adaptive immune responses can be beneficial for CNS repair. Trends Neurosci 22, 295-9 (1999b)
Schwartz M & Kipnis J. Autoimmunity on alert: naturally occurring regulatory CD4(+)CD25(+) T cells as part of the evolutionary compromise between a 'need' and a 'risk'. Trends Immunol. 23: 530-4 (2002)
Schwartz M, Shaked I, Fisher J, Mizrahi T, Schori H. Protective autoimmunity against the enemy within: fighting glutamate toxicity. Trends Neurosci. 26:297-302 (2003)
Shevah EM. Regulatory T cells in autoimmunity. Annu. Rev. Immunol. 18: 423-49 (2000)
Titelbaum D, Arnon R, Sela M. Copolymer 1: from basis research to clinical application. Cell. Mol. Life Sci. 53: 24-28 (1997)
Tenn, C.C., Fletcher, P.J. & Kapur, S. Amphetamine-sensitized animals show a sensorimotor gating and neurochemical abnormality similar to that of schizophrenia. Schizophr Res 64, 103-14 (2003)
Van den Buuse, M., Garner, B. & Koch, M. Neurodevelopmental animal models of schizophrenia: effects on prepulse inhibition. Curr Mol Med 3, 459-71 (2003)
Velakoulis, D. et al. Hippocampal volume in first-episode psychoses and chronic schizophrenia: a high-resolution magnetic resonance imaging study. Arch Gen Psychiatry 56, 133-41 (1999)
Weickert, C.S. et al. Reduced brain-derived neurotrophic factor in prefrontal cortex of patients with schizophrenia. Mol Psychiatry 8, 592-610 (2003)
Wekerle, H. Immune protection of the brain--efficient and delicate. J. Infect. Dis. 186 Suppl 2, S140-4 (2002)
Whishaw, I.Q. & Auer, R.N. Immediate and long-lasting effects of MK-801 on motor activity, spatial navigation in a swimming pool and EEG in the rat. Psychopharmacology (Berl) 98, 500-7 (1989)
Wick G, Berger P, Jansen- A Darwinian-evolutionary concept of age-related diseases.Durr, P. & Grubeck-Loebenstein B. Exp. Gerontol. 38, 13-25 (2003)
Yehuda R, Antelman SM. Criteria for rationally evaluating animal models of posttraumatic stress disorder. Biol Psychiatry 33: 479-86 (1993)
Yoles E, Hauben E, Palgi O, Agranov E, Gothilf A, Cohen A, et al. Protective autoimmunity is a physiological response to CNS trauma. J. Neurosci. 21:3740-8(2001)

## Claims

1. An agent selected from the group consisting of (i) Copolymer 1, (ii) a Copolymer 1-related peptide, (iii) a Copolymer 1-related polypeptide, and (iv) T cells activated with (i), (ii) or (iii), for use in the treatment of a psychiatric disorder, disease or condition selected from post-traumatic stress disorder (PTSD), depression, bipolar disorder and schizophrenia.

2. An agent selected from the group consisting of (i) Copolymer 1, (ii) a Copolymer 1-related peptide, (iii) a Copolymer 1-related polypeptide, and (iv) T cells activated with (i), (ii) or (iii), for use in the immunization of an individual suffering from a psychiatric disorder, disease or condition selected from post-traumatic stress disorder (PTSD), depression, bipolar disorder and schizophrenia.

3. An agent for use in the immunization according to claim 2 wherein said immunization comprises the use of an agent without an adjuvant.

4. An agent for use in the immunization according to claim 2 wherein said immunization comprises the use of an agent emulsified in an adjuvant suitable for human clinical use.

5. An agent for use in the immunization according to claim 4, wherein said adjuvant is selected from the group consisting of aluminum hydroxide, aluminum hydroxide gel, and aluminum hydroxyphosphate.

6. An agent for use in the treatment according to claim 1, or for use in the immunization according to any one of claims 2 to 5 wherein said agent is Copolymer 1.

7. An agent for use in the treatment according to claim 1, or for use in the immunization according to any one of claims 2 to 5 wherein said agent is a Copolymer 1-related peptide or a Copolymer 1-related polypeptide.

8. An agent for use in the treatment according to claim 1 wherein said psychiatric disorder disease or condition is post-traumatic stress disorder (PTSD) and said agent is Copolymer 1.

9. An agent for use in the immunization or treatment according to claim 1 wherein said psychiatric disorder disease or condition is selected from depression and bipolar disorders.

10. An agent for use in the immunization or treatment according to claim 1 wherein said psychiatric disorder, disease or condition is schizophrenia and said agent is Copolymer 1.

## Patentansprüche

1. Agens ausgewählt aus der Gruppe bestehend aus (i) Copolymer 1, (ii) einem Copolymer 1-verwandten Peptid, (iii) einem Copolymer 1-verwandten Polypeptid und (iv) T-Zellen aktiviert durch (i), (ii) oder (iii) zur Verwendung in der Behandlung einer psychiatrischen Störung, Krankheit oder Verfassung ausgewählt aus posttraumatischer Belastungsstörung (PTSD), Depression, bipolarer Störung und Schizophrenie.

2. Agens ausgewählt aus der Gruppe bestehend aus (i) Copolymer 1, (ii) einem Copolymer 1-verwandten Peptid, (iii) einem Copolymer 1-verwandten Polypeptid und (iv) T-Zellen aktiviert durch (i), (ii) oder (iii) zur Verwendung in der Immunisierung eines Individuums, welches unter einer psychiatrischen Störung, Krankheit oder Verfassung, ausgewählt aus posttraumatischer Belastungsstörung (PTSD), Depression, bipolarer Störung und Schizophrenie, leidet.

3. Agens zur Verwendung in der Immunisierung nach Anspruch 2, wobei die Immunisierung die Verwendung eines Agens ohne ein Adjuvans umfasst.

4. Agens zur Verwendung in der Immunisierung nach Anspruch 2, wobei die Immunisierung die Verwendung eines Agens umfasst, das in einem zur humanen klinischen Verwendung geeigneten Adjuvans emulgiert ist.

5. Agens zur Verwendung in der Immunisierung nach Anspruch 4, wobei das Adjuvans ausgewählt ist aus der Gruppe bestehend aus Aluminiumhydroxid, Aluminiumhydroxidgel und Aluminiumhydroxyphosphat.

6. Agens zur Verwendung in der Behandlung nach Anspruch 1 oder zur Verwendung in der Immunisierung nach einem der Ansprüche 2 bis 5, wobei das Agens Copolymer 1 ist.

7. Agens zur Verwendung in der Behandlung nach Anspruch 1 oder zur Verwendung in der Immunisierung nach einem der Ansprüche 2 bis 5, wobei das Agens ein Copolymer 1-verwandtes Peptid oder ein Copolymer 1-verwandtes Polypeptid ist.

8. Agens zur Verwendung in der Behandlung nach Anspruch 1, wobei die psychiatrische Störung, Krankheit oder Verfassung posttraumatische Belastungsstörung (PTSD) und das Agens Copolymer 1 ist.

9. Agens zur Verwendung in der Immunisierung oder Behandlung nach Anspruch 1, wobei die psychiatrische Störung, Krankheit oder Verfassung ausgewählt ist aus Depression und bipolarer Störung.

10. Agens zur Verwendung in der Immunisierung oder Behandlung nach Anspruch 1, wobei die psychiatrische Störung, Krankheit oder Verfassung Schizophrenie und das Agens Copolymer 1 ist.

## Revendications

1. Agent sélectionné dans le groupe consistant en (i) le copolymère 1, (ii) un peptide apparenté au copolymère 1, (iii) un polypeptide apparenté au copolymère 1, et (iv) des cellules T activées avec (i), (ii) ou (iii), destiné à être utilisé dans le traitement d'un trouble, d'une maladie ou d'un état pathologique psychiatrique sélectionné(e) parmi un état de stress post-traumatique (ESPT), la dépression, un trouble bipolaire et la schizophrénie.

2. Agent sélectionné dans le groupe consistant en (i) le copolymère 1, (ii) un peptide apparenté au copolymère 1, (iii) un polypeptide apparenté au copolymère 1, et (iv) des cellules T activées avec (i), (ii) ou (iii), destiné à être utilisé dans l'immunisation d'un individu souffrant d'un trouble, d'une maladie ou d'un état pathologique psychiatrique sélectionné(e) parmi un état de stress post-traumatique (ESPT), la dépression, un trouble bipolaire et la schizophrénie.

3. Agent destiné à être utilisé dans l'immunisation selon la revendication 2, où ladite immunisation comprend l'utilisation d'un agent sans adjuvant.

4. Agent destiné à être utilisé dans l'immunisation selon la revendication 2, où ladite immunisation comprend l'utilisation d'un agent émulsifié dans un adjuvant approprié pour un usage clinique humain.

5. Agent destiné à être utilisé dans l'immunisation selon la revendication 4, où, ledit adjuvant est sélectionné dans le groupe consistant en l'hydroxyde d'aluminium, un gel d'hydroxyde d'aluminium, et l'hydroxyphosphate d'aluminium.

6. Agent destiné à être utilisé dans le traitement selon la revendication 1, ou destiné à être utilisé dans l'immunisation selon l'une quelconque des revendications 2 à 5, où ledit agent est le copolymère 1.

7. Agent destiné à être utilisé dans le traitement selon la revendication 1, ou destiné à être utilisé dans l'immunisation selon l'une quelconque des revendications 2 à 5, où ledit agent est un peptide apparenté au copolymère 1 ou un polypeptide apparenté au copolymère 1.

8. Agent destiné à être utilisé dans le traitement selon la revendication 1, où ledit trouble, maladie ou état pathologique psychiatrique est un état de stress post-traumatique (ESPT) et ledit agent est le copolymère 1.

9. Agent destiné à être utilisé dans l'immunisation ou le traitement selon la revendication 1, où ledit trouble, maladie ou état pathologique psychiatrique est sélectionné(e) parmi la dépression et les troubles bipolaires.

10. Agent destiné à être utilisé dans l'immunisation ou le traitement selon la revendication 1, où ledit trouble, maladie ou état pathologique psychiatrique est la schizophrénie et ledit agent est le copolymère 1.
